# DEMANDE DE BREVET EUROPEEN

(11) **EP 4 279 170 A2**
(43) Date de publication de la demande: **22.11.2023**
(21) Numéro de dépôt: 23201377.1
(22) Date de dépôt: 02.06.2017
(51) Int. Cl.: B01F 33/84

(54) **SYSTEME DE DISTRIBUTION D'UN PRODUIT COSMETIQUE**

(30) Priorité: 02.06.2016 FR 1655053
(62) Demande divisionnaire de: 17732768.1
(71) Demandeur: L'OREAL, 75008 Paris (FR)
(72) Inventeur: GIRON, Franck, LAGNY SUR MARNE (FR); SAMAIN, Henri, BIEVRES (FR); KERGOSIEN, Guillaume, CHAVILLE (FR)
(74) Mandataire: Cabinet Nony

(57) **Abrégé**

La présente invention concerne un système (10) de distribution d'un produit comportant un distributeur recevant au moins deux cartouches (30) ayant chacune un réservoir et contenant respectivement des premier et deuxième produits de base, le premier produit de base comportant l'un au moins d'une huile, d'un pigment interférentiel ou à reflet métallique, le deuxième produit de base comportant une charge matifiante, le distributeur permettant de délivrer au moins ces deux produits de base dans des proportions réglables.

## Description

La présente invention concerne les procédés et systèmes de distribution d'un produit cosmétique, notamment d'un produit de maquillage, de coloration, de protection solaire, de soin ou d'un parfum.

De nombreuses personnes souhaitent se maquiller pour embellir leur apparence, notamment leur visage.

Les motivations de ces personnes peuvent se ranger en deux catégories :
- Masquer certaines imperfections, comme des taches, des rides ou des pores,
- Embellir le rendu du visage par des changements de couleur.

Dans ces différents cas, l'opération consiste à apporter une matière colorée et à en couvrir la peau ou une zone de peau.

Pour obtenir un effet esthétique, la personne doit réussir le choix de la matière colorée.

Dans le premier cas ci-dessus, l'opération peut être complexe car le visage comporte toute une gamme de couleurs.

Ainsi, si la personne ne veut couvrir que quelques zones du visage, en essayant de faire coïncider la couleur ajoutée à la couleur naturelle de la peau environnante, il lui est nécessaire de chercher la couleur qui convient à chaque zone du visage, ce qui est d'autant plus difficile que la couvrance du produit et l'épaisseur déposée, ainsi que la couleur et l'état de surface de la peau sous-jacente ou son caractère plus ou moins gras, peuvent influencer le résultat.

Compte-tenu de ces difficultés, les personnes qui veulent masquer les imperfections de leur visage, ont l'habitude de couvrir l'ensemble du visage. On contourne alors le problème du choix de la matière colorée en fonction de la zone du visage.

Toutefois, le résultat s'éloigne de l'aspect naturel du visage de par l'homogénéité apportée.

Dans le deuxième cas, l'opération n'est pas simple non plus car il est difficile de trouver la matière colorée qui convient le mieux à l'aspect du visage. En particulier, il est difficile de trouver la couleur de son teint, surtout si l'on veut choisir une couleur marquée, différente de sa couleur naturelle. Certaines personnes aimeraient choisir des couleurs bronzées ou autres nuances de teint différentes, mais elles ne le font pas de peur que la couleur ne leur corresponde pas bien. Et si elles le font, elles abandonnent souvent déçues. Quand elles ne sont pas déçues du résultat, elles n'osent plus changer de couleur.

Il en est de même pour le maquillage des lèvres et celui des joues et des paupières.

Dans le cas du maquillage global ou le maquillage des peaux tachées, on souhaite avoir une esthétique la plus belle possible. Or, pour parfaire l'esthétique du visage, il ne suffit pas d'appliquer la meilleure couleur au bon endroit. Ainsi, on s'aperçoit que le niveau de matité/brillance idéal peut être différent selon la zone concernée. Certaines personnes aiment que les zones comme les joues soient plutôt brillantes. A l'inverse, elles aiment que d'autres zones comme le front, soient plutôt mates

Pour résoudre ce problème, il n'existe que peu de solutions.

Une première approche consiste à acheter de nombreux produits et à tous les essayer. Cette approche est coûteuse et génère souvent des pertes dans la mesure où l'on ne retient généralement qu'un petit nombre des matières colorées essayées.

Une seconde approche consiste à tester, en magasin, différents produits. Elle ne convient pas toujours, car il est très difficile de se figurer le résultat en quelques instants et dans un lieu dépourvu des repères habituels. En particulier, pour apprécier pleinement, l'effet d'un maquillage dans un magasin, il faudrait pouvoir l'éclairer de la même manière que dans les conditions futures d'utilisation, ce qui est rarement possible. En général, ce n'est qu'en testant un maquillage pendant la journée, qu'on se rend compte s'il convient ou non. De plus, si certains magasins disposent de conseillers et permettent d'effectuer des essais, ce n'est pas le cas d'un grand nombre d'autres points de vente et des achats effectués sur internet.

Une autre approche a été testée sans toutefois se développer. Elle consiste à réaliser manuellement ses produits par mélange de plusieurs produits de couleur. Cela peut s'avérer relativement difficile à mettre en oeuvre, car il est peu aisé de reproduire exactement plusieurs fois le même mélange, et l'on a du mal à réaliser rapidement les mélanges dont on a besoin au moment du maquillage.

On connaît aussi des distributeurs permettant de délivrer une composition cosmétique de couleur variable.

La demande US2003069667 concerne des procédés et des appareils permettant de personnaliser les produits cosmétiques utilisés par un consommateur. Ce dernier fournit des critères de choix et une formule de produit cosmétique en est dérivée. Les ingrédients de base sont mélangés conformément à la formule et un produit cosmétique personnalisé est distribué sur une surface intermédiaire, pour une application ultérieure.

Le brevet US5785960 divulgue un procédé d'obtention de fonds de teint adaptés à recouvrir les imperfections de la peau humaine. Les étapes du procédé incluent la mesure par spectrophotomètre d'une peau normale d'un client pour obtenir des valeurs de brillance, de rouge et de jaune de coloration de la peau, respectivement dénotées en tant qu'unités L, a et b. Ensuite ces valeurs sont converties par calcul en des valeurs modifiées déterminées par un programme de correction des valeurs L, a et b. En fonction de ces valeurs modifiées, un fond de teint est formulé. Une machine de formulation à distance convertit les instructions reçues et dose et mélange une série de produits de base. Le mélange délivré par la machine est emballé et expédié au client.

La demande FR2970403 divulgue un dispositif de distribution d'un produit cosmétique, notamment d'un parfum, comportant au moins un réservoir contenant un produit à distribuer, notamment plusieurs réservoirs, et un dispositif de rinçage. Le dispositif peut être piloté à l'aide d'un micro-ordinateur ou similaire. Une interface homme machine, par exemple un clavier ou un écran, notamment tactile, permet à l'utilisateur de commander la distribution d'une formule de son choix. Le dispositif peut être agencé pour communiquer avec un serveur ou d'autres dispositifs similaires pour échanger des recettes ou permettre à l'utilisateur de se faire conseiller. Une mémoire d'un circuit électronique du dispositif peut enregistrer les meilleures formules, afin de les reproduire à la demande et de les échanger. Le dispositif peut aussi être utilisé pour réaliser des mélanges de produits cosmétiques colorés. Une certaine quantité, par exemple une goutte, de composition colorée est alors produite par le dispositif et sert à se maquiller ou est à mélanger à une crème de teint ou toute autre base colorée ou non. Le dispositif permet de générer facilement la couleur voulue par l'utilisateur, lequel peut par exemple réaliser en quelques instants plusieurs mélanges de couleurs différentes.

La demande de brevet FR2818101 concerne un dispositif pour la pulvérisation d'un produit cosmétique, notamment d'un fond de teint. Il est possible de réaliser un mélange extemporané sur le substrat traité.

La demande FR 2877819 décrit un distributeur permettant de faire varier la proportion relative de différents produits de base qui sont distribués. Il est ainsi possible de régler la couleur. Les produits de base sont issus de différents réservoirs et distribués par des canaux distincts qui débouchent côte à côte à une extrémité du distributeur. Un inconvénient qui en résulte est que l'utilisateur doit effectuer le mélange sur la peau ou sur un support intermédiaire. De plus, si la quantité distribuée est excessive, celle-ci est perdue.

US 5 622 692 et US5903465 décrivent d'autres exemples de distributeurs permettant de distribuer une composition cosmétique personnalisée.

Parmi les essais d'automatisation de la fabrication d'une composition cosmétique personnalisée qui ont pu être tentés, nombreux sont ceux dans lesquels les solutions proposées permettent de réaliser des mélanges dans des quantités d'environ 100 g ou parfois moins, mais pas dans les très petites proportions dont la personne qui se maquille a généralement besoin, c'est-à-dire de l'ordre du gramme ou beaucoup moins.

Pour illustrer cette problématique, on peut imaginer le cas d'une personne qui veut maquiller son visage avec un dégradé de matité/brillance allant du mat au brillant parant du nez (mat) jusqu'aux joues (brillantes) puis jusqu'au bord du visage (mat). Pour réaliser cet effet, il faut qu'elle puisse appliquer plusieurs touches de produit, de même couleur mais avec des niveaux de matité/brillance différents et commandables. Deux produits ne suffiront pas car produisant alors des démarcations entre les zones. Ainsi, il y a un besoin de disposer rapidement et de façon sure des petites quantités de produit avec matité/brillance variables. Les touches peuvent être par exemple de l'ordre des 10-50 mg par touche et servir à ne couvrir que des zones d'environ quelques cm2. Le même exercice est encore plus difficile si, pour éviter une sorte d'uniformité, la personne voudra faire varier la couleur (couleur et couvrance) tout en faisant varier le niveau de matité/brillance.

L'invention vise ainsi, selon certains de ses aspects, à faciliter le maquillage du visage et notamment la recherche des produits les plus adaptés en terme de matité/brillance aux différentes zones de celui-ci et l'obtention rapide d'un résultat avec des niveaux de matité/brillance adaptées aux différentes zones.

L'invention vise aussi à obtenir une couleur donnée, avec différents niveaux de matité/brillance.

L'invention repose sur un système de distribution qui permet de générer des mélanges à partir de produits de base. Ces produits de base peuvent avoir des couleurs différentes (ou pas de couleur), de telle sorte que l'on puisse faire varier la matité/brillance du mélange. Les produits de base peuvent encore permettre de faire varier la couvrance et/ou couleur du mélange, de telle sorte que la couleur qui résulte de l'application du mélange sur les matières kératiniques humaines varie, en étant plus ou moins proche de celle desdites matières.

Ainsi, l'invention permet de fixer une couleur et/ou brillance et faire varier la matité/brillance ou faire varier l'ensemble. La notion de couleur doit être comprise avec une acceptation large et englobe des mélanges dont la couleur varie après application du fait de leur couvrance plus ou moins élevée et de la couleur de la peau sous-jacente.

L'invention a ainsi pour objet un système de distribution d'un produit, comportant un distributeur recevant au moins deux cartouches ayant chacune un réservoir et contenant respectivement des premier et deuxième produits de base, le premier produit de base comportant l'un au moins d'une huile, d'un pigment interférentiel ou à reflet métallique, le deuxième produit de base comportant une charge matifiante, le distributeur permettant de délivrer au moins ces deux produits de base dans des proportions réglables.

L'invention peut présenter l'une ou plusieurs des caractéristiques préférentielles suivantes :
- le premier produit de base contient une huile,
- le premier produit de base contient un pigment interférentiel,
- le premier produit de base contient un pigment à reflet métallique,
- la taille D50 en volume de particule du pigment et de la charge est comprise entre 100 nm et 1 mm, mieux entre 200 nm et 100 microns, encore mieux entre 200 nm et 50 microns,
- la charge est choisie parmi le talc, le mica, la silice, le kaolin, les poudres de poly-β-alanine et de polyéthylène, les poudres de polymères de tétrafluoroéthylène, les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges, préférentiellement est choisie parmi le talc, le mica, la silice, les poudres de nylon, les poudres de polymethacrylate de methyle, et leurs mélanges, encore préférentiellement est du talc,
- la charge peut comporter un enrobage comportant au moins un composé lipophile ou hydrophobe,
- le premier produit de base et le deuxième produit de base comportent une émulsion inverse,
- la teneur massique en charge dans le deuxième produit de base est supérieure ou égale à 0,5% de la masse du premier produit de base, de préférence supérieure à 1% de la masse du premier produit de base, encore de préférence comprise entre 1% et 5% de la masse du premier produit de base,
- le système de distribution comporte une troisième cartouche avec un troisième produit de base,
- les cartouches sont reçues de façon amovible dans le distributeur,
- chaque produit de base quitte la cartouche par un canal de sortie de la cartouche, le canal de sortie étant défini par un embout de distribution de la cartouche entraîné en rotation relative par rapport à un corps de la cartouche par un mécanisme d'entraînement du distributeur pour distribuer le produit de base contenu dans la cartouche.

L'invention a encore pour objet un procédé d'application d'un produit cosmétique, de maquillage et/ou de soin, sur les matières kératiniques humaines, comportant le réglage du distributeur en fonction de la zone à traiter, et la distribution du produit par prélèvement dans les cartouches des produits de base dans les proportions correspondant au réglage du distributeur.

L'invention repose ainsi sur un système de distribution, avec des compartiments contenant au moins deux formulations différentes, lesquelles contiennent de préférence des émulsions inverses concentrées pour l'une en huiles, nacres, ou particules à reflet métallique, et concentrée pour l'autre en charges (matifiantes).

On peut donc faire varier le niveau de matité/brillance tout en gardant un aspect coloriel égal.

On peut programmer le système pour qu'il délivre pour chaque endroit du visage le meilleur mélange matifiant ou brillant. L'aspect global ne permettra pas de deviner que le visage a été traité car permettant de se rapprocher de l'aspect d'un visage naturel.

### Système de distribution

Le système de distribution peut être constitué par un seul appareil fonctionnant de manière autonome, de préférence manipulable d'une main, ou par un appareil qui fonctionne en interagissant avec d'autres pièces ou appareils. Il peut s'agir par exemple de différentes interfaces de sorties qui sont montées sur le distributeur en fonction du type de maquillage à réaliser, comme cela sera précisé plus loin. Il peut s'agir également d'un système informatique qui échange des informations avec le distributeur pour le piloter, ce système informatique comportant par exemple un terminal portable tel qu'un téléphone intelligent, un photophone, une tablette, un ordinateur personnel, ou un terminal dédié.

De préférence, le distributeur est agencé pour mettre sous pression un ou plusieurs compartiments contenant le ou les produits de base, par le biais de dispositifs de dosage volumétrique, de préférence un moteur entraînant en déplacement un piston dans le compartiment correspondant.

Le distributeur peut être formé d'un boîtier et d'au moins deux ou trois compartiments, et de préférence d'autant de moteurs. Par exemple, la rotation des moteurs entraîne des vis sans fin, lesquelles poussent les pistons de chaque compartiment. L'avance des pistons est par exemple contrôlée par le nombre d'impulsions de commande envoyées aux moteurs et/ou par la durée de fonctionnement de ces derniers. Les moteurs peuvent être alimentés séquentiellement, ou de préférence simultanément.

Par exemple, les moteurs sont alimentés durant un cycle élémentaire de fonctionnement pendant une courte durée les uns après les autres, ou les uns en même temps que les autres, de façon à distribuer des microdoses correspondantes.

Les cycles élémentaires sont répétés, avec éventuellement un temps d'arrêt entre eux, qui laisse le temps aux produits de base de s'écouler hors des compartiments.

Les compartiments peuvent être définis par des cartouches qui se retirent lorsqu'elles sont vides. En variante, les compartiments sont présents à demeure et remplis à nouveau une fois vide.

Chaque cartouche peut être fermée par un bouchon démontable pour permettre le nettoyage de la cartouche.

De préférence, le boîtier du distributeur est de forme allongée selon un axe longitudinal, ce qui peut faciliter sa manipulation, et les cartouches sont disposées autour de cet axe, dans le boîtier.

De préférence, les cartouches sont mises en place par l'arrière, et le mélange est délivré par l'avant. La mise en place des cartouches peut s'effectuer individuellement, ou en variante les cartouches constituent un ensemble monobloc lors de leur mise en place.

Les cartouches peuvent comporter chacune un mécanisme de dosage volumétrique, comportant un piston mu par un mécanisme d'entraînement du distributeur, dans une direction s'accompagnant d'une diminution du volume intérieur contenant le produit de base et d'une expulsion du produit. Il peut être intéressant que les cartouches aient une région de leur paroi au moins qui soit transparente, afin de permettre de voir la couleur du produit contenu à l'intérieur.

Le mécanisme d'entraînement peut comporter un système de motorisation formé de moteurs couplés à des réducteurs, de forme allongée parallèlement à l'axe longitudinal du distributeur, et disposés entre les cartouches. Ce positionnement des moteurs et cartouches rend le distributeur particulièrement compact.

Le produit de base peut sortir de façon étanche de la cartouche correspondante puis circuler dans un canal prévu à cet effet dans le boîtier du distributeur, avant de sortir de celui-ci.

Les cartouches se terminent avantageusement par un embout réalisé de telle sorte que, une fois la cartouche insérée dans le boîtier du distributeur, l'extrémité de l'embout débouche au ras du boîtier. En variante, l'embout est suffisamment long pour dépasser du boîtier et connecter ainsi différentes interfaces de sortie susceptibles d'être rapportées sur le boîtier du distributeur.

Grâce au mécanisme d'entraînement comportant des moteurs pour faire avancer des pistons, il est possible de délivrer de façon précise des mélanges en de très petites quantités. Ainsi, le mécanisme d'entraînement peut délivrer les produits de base avec à un débit minimal inférieur ou égal à 50 µL/s, mieux inférieur ou égal à 20 µL/s, encore mieux inférieur ou égal 10 µL/s. De préférence, le mécanisme d'entraînement délivre des débits compris entre 20 et 100 µL/s, mieux compris entre 40 µL/s et 60 µL/s. Il est donc possible de réaliser facilement un mélange d'environ 10 mg. Un tel système de distribution est alors idéal pour réaliser de petites touches de maquillage, pour couvrir par exemple une zone de 1 cm², mieux une zone de 0, 5 cm².

Il est aussi possible de réaliser des plus grandes quantités de mélange comme celles nécessaires au maquillage d'une joue, ou d'un visage. Ces quantités restent néanmoins relativement faibles, par exemple une quantité comprise entre 100 et 500 mg, mieux comprise entre 150 et 250 mg.

Le distributeur présente de préférence un boîtier, et au moins une cartouche reçue dans le boîtier du distributeur, cette cartouche comportant un corps et un piston mobile dans le corps, le boîtier comportant un mécanisme d'entraînement motorisé pour déplacer le piston de la cartouche.

De préférence, la cartouche comporte un embout de distribution par lequel s'effectue la sortie du produit, et cet embout de distribution est entraîné en rotation par le mécanisme d'entraînement pour déplacer le piston. L'embout peut comporter au moins un relief d'anti-rotation, mieux deux ergots d'anti-rotation diamétralement opposés.

L'embout peut porter un joint d'étanchéité, notamment un joint torique. Ainsi, au changement de cartouche, le joint est également changé, ce qui permet de s'affranchir de l'usure du joint.

Le distributeur peut comporter une carte électronique de pilotage du mécanisme d'entraînement motorisé, cette carte électronique étant traversée par le ou les embouts. Cela peut permettre de réaliser une carte s'étendant sensiblement sur toute la section du distributeur, donc de regrouper sur une seule carte tous les composants électroniques du distributeur, et ainsi de gagner en compacité et fiabilité. La carte peut s'étendre sensiblement perpendiculairement à l'axe longitudinal du boîtier. La carte peut porter un interrupteur de commande du fonctionnement du distributeur.

Le système de distribution peut être agencé pour fonctionner selon au moins deux modes de distribution.

Dans un premier mode, dit « continu », le mélange est distribué tant qu'une pression est exercée sur l'interrupteur de commande.

Dans un deuxième mode, dit « dose », une quantité prédéfinie du mélange est distribuée à chaque pression sur l'interrupteur.

Le ou les embouts peuvent déboucher à une extrémité du boîtier. Cela peut permettre de réduire le volume mort, comme cela sera détaillé dans la suite.

Le ou les embouts peuvent présenter à leur extrémité un système d'obturation pour éviter le séchage des produits dans le conduit, par exemple une membrane cicatrisante

La cartouche peut comporter une vis creuse sur laquelle le piston est vissé, le piston pouvant se déplacer axialement sur la vis lorsque celle-ci tourne ; le piston est empêché de tourner dans le corps de la cartouche. Par exemple, la friction du piston sur le corps de la cartouche peut suffire à l'empêcher de tourner quand la vis tourne.

Préférentiellement, la rotation est rendue impossible avec un corps de cartouche de section non circulaire et un piston non déformable.

Le couple des moteurs peut être déterminé électroniquement en fonction du courant absorbé, et servir par exemple à détecter une fin de course du piston. Une information concernant le couple peut être transmise à distance à un système informatique présentant une interface homme machine, pour permettre de surveiller le bon fonctionnement du distributeur.

Afin d'ajuster la teinte, le système de distribution selon l'invention doit permettre à l'utilisateur de faire varier le volume délivré provenant de chaque compartiment.

De façon préférée, le distributeur est piloté par un système informatique, qui est intégré au distributeur, ou externe à celui-ci, le distributeur étant alors apte à échanger avec le système informatique des informations par protocole sans fil ou câblé.

Le distributeur peut ainsi être piloté pour permettre l'ajustement de la teinte par distribution simultanée ou séquentielle, et contrôlée, de plusieurs produits de base de couleurs différentes.

La distribution des produits de base peut être continue ; si dans ce cas, les volumes de chacun des produits de base sont distribués d'une seule traite, simultanément ou successivement.

Dans le cas d'une distribution simultanée, il est utile de pouvoir ajuster les débits respectifs des différents produits de base pour que le mélange distribué corresponde à celui souhaité à chaque instant. Un tel mode de distribution peut convenir en particulier lors de la distribution du mélange par pulvérisation, à l'aide d'un aérographe. Pour ajuster les débits il est possible par exemple de jouer sur la vitesse de déplacement des pistons, par exemple, dans le cas d'un entrainement des pistons par une vis sans fin en faisant varier la vitesse de rotation des moteurs qui entrainent la vis. Les produits peuvent aussi être distribués de façon impulsionnelle avec un temps de distribution et un temps d'arrêt à chaque cycle. En jouant sur le rapport cyclique, il est possible d'agir sur le débit.

Tous les produits peuvent être délivrés simultanément lors du temps de distribution ; en variante, les cycles des différents produits sont déphasés, de sorte qu'un produit est distribué pendant un temps d'arrêt des autres produits.

Dans un mode particulier de l'invention, le mélange est délivré dans une cavité d'un contenant, pouvant fermer de façon hermétique ou non, par exemple en forme de coupelle, dans lequel on peut faire glisser un applicateur, en particulier un stylet ou une brosse.
- Lorsqu'il n'est pas amovible, le contenant peut être réalisé avec le corps du distributeur. Lorsqu'il est amovible, il peut constituer une interface de sortie parmi d'autres pouvant être montées sur le distributeur.

Les compartiments, et en particulier les cartouches, peuvent contenir tout ou partie du mécanisme d'entraînement, et par exemple le système de motorisation, ou mieux, une partie du système de motorisation, et ce, afin de réduire le nombre de pièces en mouvement dans le corps du distributeur à l'extérieur des cartouches. Par exemple, les cartouches comportent le rotor du moteur. Une fois les cartouches installées dans le corps du distributeur, les rotors sont mis en interaction avec les stators.

Le système de distribution est avantageusement agencé pour permettre de lancer des séquences préprogrammées dans lesquelles le mélange délivré par celui-ci est modifié de façon continue ou discontinue. Un mode « dégradé » permet par exemple passer progressivement d'un mélange A à un mélange B. Dans le cas d'une application par pulvérisation, notamment à l'aide d'un aérographe, cela permet de réaliser simplement les dégradés. Un mode « Alternatif » permet par exemple de passer rapidement d'un mélange

A à un mélange B plusieurs fois de suite. Dans le cas d'une application par pulvérisation, on peut ainsi réaliser un dépôt multicouche, avec des formulations différentes pour deux couches adjacentes superposées. Un autre mode permet par exemple de proposer plusieurs mélanges successifs préprogrammés, le système informatique indiquant à chaque fois l'usage qu'il faut en faire à l'utilisateur, par exemple par affichage sur un écran.

Dans le cas d'une application manuelle, les mélanges sont distribués par exemple dans une coupelle. La personne se maquille à l'endroit recommandé avec un mélange correspondant prélevé dans la coupelle, puis nettoie éventuellement la coupelle et commande la délivrance d'un nouveau mélange ; l'opération est répétée autant de fois que nécessaire pour se maquiller complètement.

L'homogénéisation des mélanges distribués peut se faire de différentes façons selon les cas d'utilisation. Dans le cas d'une application manuelle, elle peut se faire directement sur la zone d'application au moment de l'application ou dans la coupelle avant prélèvement ; dans le cas d'une application à l'aérographe, c'est la tuyère de l'aérographe qui sert de chambre de mélange ; dans le cas où le mélange est distribué dans un contenant en vue d'une utilisation ultérieure, l'homogénéisation peut être faite à la main ou en faisant passer les produits distribués dans une chambre de mélange, située entre le distributeur et le contenant ou directement intégrée au contenant, comme détaillé par la suite.

Le produit peut être délivré par le système de distribution et utilisé extemporanément. En variante, le produit délivré par le système de distribution est conditionné et utilisé ultérieurement, par exemple à plusieurs reprises, avec par exemple un jour d'intervalle au moins entre deux utilisations.

L'invention repose sur un système de distribution, des compartiments contenant au moins deux formulations différentes lesquelles peuvent contenir des émulsions inverses concentrées pour l'une en huiles, nacres, ou particules à reflet métallique, et concentrée pour l'autre en charges (matifiantes).

### Huiles

Par « huiles », on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C4 à C24, ces dernières pouvant être linéaires ou ramifiées, saturées ou insaturées; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique,

- les éthers de synthèse ayant de 10 à 40 atomes de carbone;
- les hydrocarbures linéaires ou ramifiés, d'origine minérale ou synthétique tels que la vaseline, les polydécènes, le polyisobutène hydrogéné, le squalane, les huiles de paraffine, et leurs mélanges,
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, les benzoates d'alcools en C12 à C15, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

De manière préférentielle, la phase huileuse comprend au moins une huile siliconée et encore plus préférentiellement choisie parmi
- les huiles volatiles siliconées cycliques ayant une viscosité à température ambiante inférieure à 8 cSt et ayant notamment de 4 à 7 atomes de silicium, ces silicones comportant éventuellement des groupes alkyle ou alcoxy ayant de 1 à 10 atomes de carbone, en particulier choisie parmi l'hexamethylcyclotrisiloxane, l'octamethylcylotetrasiloxane, le decamethylcyclopentasiloxane et le dodecamethylcyclohexasiloxane (cyclohexasiloxane), et leurs mélanges ;
- les polydiméthylsiloxanes (PDMS) volatiles ou non volatiles (Nom INCI : DIMETHICONE) ;
- les silicones phénylées ;
- les polydiméthylsiloxanes comprenant des groupes aliphatiques, en particulier alkyle, ou alcoxy, qui sont pendants et/ou à la fin de la chaîne silicone ; ces groupes comprenant chacun de 6 à 24 atomes de carbone, et plus particulièrement le caprylyl methicone tel que le produit commercial DOW CORNING FZ-3196^{®} par la société DOW CORNING ;
- leurs mélanges.

### Pigment interférentiel (Nacres)

Par « pigment interférentiel » encore appelé « *nacres* », il faut comprendre des particules colorées de toute forme, irisées ou non, notamment produites par certains mollusques dans leur coquille ou bien synthétisées, et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Les nacres peuvent plus particulièrement posséder une couleur ou un reflet jaune, rose, rouge, bronze, orangé, brun, or et/ou cuivré.

De manière avantageuse, les nacres conformes à l'invention sont les micas recouverts de dioxyde de titane ou d'oxyde de fer ainsi que l'oxychlorure de bismuth.

### Particules à reflet métallique

Par « *particules à reflet métallique* », au sens de la présente invention, on entend tout composé dont la nature, la taille, la structure et l'état de surface lui permet de réfléchir la lumière incidente notamment de façon non iridescente.

Les particules à reflet métallique utilisables dans l'invention sont en particulier choisies parmi :
- les particules d'au moins un métal et/ou d'au moins un dérivé métallique ;
- les particules comportant un substrat, organique ou minéral, monomatière ou multimatériaux, recouvert au moins partiellement par au moins une couche à reflet métallique comprenant au moins un métal et/ou au moins un dérivé métallique ; et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « *dérivés métalliques* »*,* on désigne des composés dérivés de métaux notamment des oxydes, des fluorures, des chlorures et des sulfures.

### Charges

Ces charges sont des particules incolores ou blanches, solides de toutes formes, qui se présentent sous une forme insoluble et dispersée dans le milieu de la composition.

A titre illustratif de ces charges, peuvent être cités le talc, le mica, la silice, le kaolin, les poudres de poly-β-alanine et de polyéthylène, les poudres de polymères de tétrafluoroéthylène (Téflon^{®}), les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique, les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique. On peut également utiliser des particules, qui ont la forme de portions de sphères creuses, telles que décrites dans les demandes de brevet JP-2003 128 788 et JP-2000 191 789.

Les charges peuvent comporter un enrobage comportant au moins un composé lipophile ou hydrophobe.

### Emulsions inverses / Compositions anhydres

Selon l'invention, lorsqu'il est précisé qu'une composition est sous la forme d'une émulsion inverse, il est entendu qu'elle peut également être alternativement sous une autre forme selon la composition considérée.

Un produit de base peut se présenter sous la forme d'une émulsion, par exemple une émulsion H/E, E/H, H/E/H ou E/H/E, et de préférence une émulsion inverse E/H, ou sous la forme d'une composition anhydre, comprenant notamment des composés carbonés et/ou siliconés, tels que des huiles hydrocarbonées et/ou siliconées.

Les émulsions selon l'invention sont de préférence des émulsions eau-dans-huile (E/H) encore appelées émulsions inverses, à savoir constituées d'une phase huileuse continue dans laquelle est dispersée la phase aqueuse sous forme de gouttelettes de manière à observer un mélange macroscopiquement homogène à l'oeil nu.

### Utilisation pour le maquillage d'une ou plusieurs zones précises de peau

Le système de distribution permet de se maquiller jour après jour, en ne traitant que les zones à masquer. Pour ce faire, on délivre de petites doses de maquillage, qu'on applique précisément et séquentiellement sur les zones correspondantes. Chaque petite dose est réalisée selon le mélange qui convient à la zone.

Dans un mode préféré de mise en oeuvre de l'invention, le système de distribution attend qu'on le renseigne sur une zone à traiter puis délivre le mélange correspondant. Il peut faire appel à une table de correspondance préprogrammée pour cela, cette table résultant par exemple d'un procédé d'apprentissage tel que défini plus loin. En variante, le système de distribution renseigne la personne, tout en délivrant un mélange, sur la zone sur laquelle celle-ci doit appliquer le mélange. Ainsi, le système de distribution peut suivre un programme d'application où il délivre dans un ordre donné, les différents mélanges à appliquer.

Dans un mode particulier de mise en oeuvre de l'invention, le système de distribution est renseigné sur les quantités à délivrer. Pour cela, il mémorise la correspondance entre la matité/brillance, la zone du visage et la quantité nécessaire, ce qui permet de réduire les coûts et les pertes de produit, et de ne couvrir que faiblement la peau, évitant ainsi des effets d'occlusion. Ce faisant, il est possible d'utiliser des produits très matifiants et trop matifiants ou très brillants ou trop brillants pour être appliqués sur l'ensemble du visage. Ainsi, il est possible d'obtenir des maquillages d'aspects naturels, voire indétectables.

Le système de distribution peut aussi permettre, en facilitant la distribution de petites quantités et leur usage rapide, de réduire la durée de conservation des produits, permettant ainsi de réduire les risques d'évolution et/ou les quantités de conservateurs à employer.

Le système de distribution est adapté à traiter les zones qu'on veut masquer, sans avoir à masquer l'ensemble du visage.

Dans une variante spécialement avantageuse, le système peut être utilisé pour application sur le fond du visage une formule matte puis une ou deux applications sur des zones précises du visage des formules plus brillantes. On peut aussi commencer par applications sur des zones précises du visage des formules brillantes puis appliquer sur le fond du visage une application d'une formule plus matte.

Dans le second cas, on prend le temps, avant d'appliquer la seconde couche, d'attendre que le film produit par l'application sur les zones précises devienne suffisamment cohésif pour que la seconde couche n'emporte pas la première couche.

Ou bien, on applique la seconde couche sans frotter (spray par exemple, ou éponge).

Lorsque l'utilisateur recherche la matité/brillance à appliquer sur une zone du visage, il est intéressant de mémoriser la matité/brillance qui va bien pour chaque zone, et le système de distribution est ainsi avantageusement agencé pour mémoriser cette matité/brillance et la zone correspondante. Ainsi, en utilisant les informations mémorisées, à chaque utilisation, on peut obtenir la délivrance du même mélange pour chaque zone, ou, si l'on traite plusieurs zones, la même série de mélanges pour une même série de zones.

Le système de distribution peut aussi être agencé pour permettre de traiter une zone en faisant varier les matité/brillances application après application. Ainsi, la personne peut maquiller les lèvres avec différentes couleurs, qu'elle choisit au jour le jour selon ses goûts. Cette approche est aussi adaptée aux paupières, ainsi qu'au maquillage du visage car la personne peut avoir envie de changer de couleur de teint. Par exemple, les jours de la semaine, la personne réalise un fond de teint couleur plutôt mat, et le weekend, un fond de teint de plutôt brillant.

Le système de distribution peut être agencé pour permettre à l'utilisateur de changer de matité/brillance en plus des paramètres couleurs/couvrance selon ses goûts du jour, du moment, de ses tenues, du climat. Ainsi, on prévoit avantageusement un système d'aide à la décision pour guider l'utilisateur dans ses choix de matité/brillance et/ou couleur (au sens large).

On peut prévoir aussi un système d'aide pour équilibrer les matité/brillance sur un même visage et participer à la réalisation d'un maquillage global réussi.

Il peut être souhaitable que plusieurs personnes d'un même groupe, par exemple une famille, puissent utiliser le système de distribution, réduisant ainsi les coûts et minimisant la place occupée. Cette solution est spécialement adaptée aux voyages ou aux hôtels, campings, avions, campings cars, boutiques, écoles... Pour ce faire, on peut prévoir que le système de distribution puisse être renseigné sur la personne qui l'utilise, afin d'accéder à des données personnelles préalablement mémorisées.

### Utilisation en continu pour un maquillage en dégradé de matité/brillance

Dans cette application, le système de distribution, pendant qu'il délivre le produit, change la formulation du mélange. De plus, on déplace la sortie des produits de base ou du mélange relativement à un contenant ou un support définissant une surface d'application. Dans un mode particulier de mise en oeuvre de l'invention, le système de distribution est agencé pour calculer l'évolution du mélange en fonction de la matité/brillance C1, d'une zone à traiter et de la matité/brillance C2, d'une autre zone à traiter. Par exemple, sachant que le menton nécessite une matité/brillance C1 et que la joue nécessite une matité/brillance C2, le système de distribution peut faire varier la formulation du mélange pendant qu'il le délivre pour réaliser un dégradé de matité/brillance entre ces deux points. Cela permet par exemple de mieux masquer les imperfections du visage en faisant en sorte que le résultat final soit réaliste, ou de faire des dégradés de matité/brillance à des fins d'embellissement. Le système de distribution peut encore être agencé de telle sorte que l'utilisateur puisse commander une variation de matité/brillance du mélange distribué sans que les matité/brillance de départ et/ou d'arrivée n'aient été préalablement fixées. Pour ce faire, le système de distribution peut disposer d'un système de localisation, ou d'auto-localisation, et déduire d'une table de correspondance les matité/brillance C1 et C2 qu'il doit réaliser, et donc les évolutions de mélange qu'il doit réaliser.

Le système de distribution peut comporter une tête de sortie, en particulier dans le cas d'un aérographe, qui est mobile et pilotée. Cette option permet alors, sans bouger le reste du système de distribution, de réaliser des dégradés. Par exemple, on localise le système de distribution près de la joue, puis on déclenche un système de commande qui va piloter automatiquement la variation de la formulation du mélange et le mouvement de la tête de sortie, de façon à par exemple avoir le centre de la joue plus rouge que sa périphérie, avec un dégradé entre les deux.

Le système de distribution peut encore être utilisé pour réaliser des produits sur mesure, que l'on conserve pour réaliser plusieurs applications.

Il est aussi possible de réaliser des produits solides ou semi-solides.

### Fabrication de compacts « à façon » ou autres produits solides ou semi-solides

Le système de distribution peut être agencé pour permettre de choisir un mélange et de le délivrer dans un contenant tel qu'une coupelle. Le mélange comporte de préférence des composés qui sont tels que le mélange puisse prendre en masse.

Plus préférentiellement, on utilise des composés qui rendent la prise en masse spécialement rapide. Ces composés sont soit déposés dans le contenant avant ou après son remplissage avec les autres constituants, soit sont prévus dans les compartiments du distributeur avec les autres constituants des produits de base, soit sont compris dans le distributeur dans un compartiment spécialement prévu pour les contenir.

On peut ainsi distribuer des compositions spécifiques qui peuvent par réaction chimique, biochimique ou physicochimique, durcir de façon accélérée après évacuation.

Ces compositions sont spécialement adaptées à la réalisation des compacts, c'est-à-dire :
- Prennent en masse,
- donnent un matériau qui peut se déliter en cas de frottement, et sont préférentiellement colorées.

De préférence, ces compositions sont très riches en particules solides, avec par exemple plus de 10% en masse de particules solides par rapport au poids total de la composition, mieux plus de 20% en masse de particules solides par rapport à la masse totale de la composition, encore mieux plus de 30% en masse de particules solides par rapport à la masse totale de la composition, préférentiellement entre 10 et 40% en masse de particules solides par rapport à la masse totale de la composition.

Ces compositions peuvent contenir des particules absorbantes, ou des composés réactifs, tels que ceux qui réagissent en contact avec l'air, par exemple du cyanoacrylate ou des alphasilanes, ou qui réagissent à la lumière, notamment UV.

Le contenant dans lequel le mélange est distribué peut comporter un composé A et les compositions distribuées un composé B, A et B étant choisis pour réagir entre eux et solidifier le mélange.

Dans un mode particulier de mise en oeuvre de l'invention, le système de distribution intègre un moyen de chauffage, par exemple à résistance électrique, pour la réalisation de rouges à lèvres ou autres produits cireux. Dans ce cas, les produits de base sont chauffés avant d'être délivrés.

Le système de distribution peut aussi comporter un moyen pour apporter de l'énergie, thermique et/ou lumineuse, après distribution du mélange dans un contenant, par exemple une résistance électrique ou une diode LED, notamment UV. Cette énergie peut accélérer la prise en masse du mélange distribué.

Préférentiellement, le mélange est homogénéisé avant la prise en masse.

### Réalisation de palettes de matité/brillance

Le système de distribution peut comporter un support, ayant plusieurs régions, et être agencé pour générer automatiquement plusieurs mélanges déposés dans lesdites régions, par exemple une série de matité/brillance adaptées à différentes parties du visage.

Le support peut définir plusieurs cavités pour recevoir les mélanges ou porter plusieurs contenants, par exemple sous forme de coupelles, éventuellement séparables du support.

Dans un cas particulier, le support prend la forme d'un visage avec des régions pour recevoir les mélanges pour des zones d'applications ciblées.

Le support être mobile, notamment rotatif, par rapport au corps du distributeur, et par exemple entraîné en déplacement par le distributeur, pour remplir successivement différents logements ou contenants.

Les produits, sur la palette pourront être de couleurs différentes, tout en présentant des effets de matité/brillance différentes

### Distributeur à coupelle

Il existe un intérêt pour bénéficier d'un système de distribution capable de délivrer un mélange pouvant être facilement prélevé par l'utilisateur. Par ailleurs, dans le cas où les produits de base délivrés par le système de distribution ne sont pas déjà mélangés, il existe un besoin pour permettre à l'utilisateur d'effectuer aisément le mélange.

L'invention a encore pour objet un système de distribution d'au moins un produit de maquillage, comportant une coupelle, et un distributeur pour remplir la coupelle avec au moins un produit, la coupelle étant solidaire du distributeur au moins pendant son remplissage.

La coupelle est parfois encore appelée « creuset » et ce terme doit être compris avec une acceptation large.

Par « coupelle solidaire du distributeur » il faut comprendre que la coupelle est retenue, notamment immobilisée, au moins temporairement sur le distributeur, étant par exemple fixée sur celui-ci par des vis, par aimantation, par encliquetage, par verrouillage baïonnette, par serrage, ou réalisée avec une partie du corps du distributeur par moulage de matière. La coupelle lorsqu'elle est solidaire du distributeur permet de manipuler celui-ci d'une main, la coupelle restant en place sur le distributeur au cours des mouvements de ce dernier.

Le distributeur peut être proposé à l'utilisateur avec la coupelle déjà en place.

En variante, celle-ci est installée par l'utilisateur à la première utilisation du système de distribution.

De préférence la coupelle est moins profonde que large, ce qui facilite l'accès à celle-ci, et permet de prélever le produit, notamment le mélange avec un applicateur ou le doigt.

De préférence, la coupelle est séparable du distributeur, et constitue une interface de sortie qui peut être choisie parmi un ensemble d'interfaces de sortie pouvant être montées sur le distributeur, au choix de l'utilisateur en fonction du maquillage à réaliser, comme détaillé plus loin.

De préférence, le système de distribution comporte plusieurs orifices de remplissage en des produits de base différents, débouchant dans la coupelle. Ainsi, le mélange de ces produits peut avoir lieu dans la coupelle.

La coupelle a de préférence un fond de forme concave vers l'extérieur, ce qui peut faciliter son nettoyage par l'utilisateur entre deux utilisations.

De plus, cela peut faciliter le prélèvement du produit par l'utilisateur, et le mélange des produits de base.

De préférence, le distributeur permet de délivrer au moins deux produits de base dans la coupelle, dans des proportions réglables, et mieux au moins trois produits.

Dans un exemple de mise en oeuvre, le système de distribution comporte au moins deux coupelles pouvant être sélectivement alimentées par le distributeur. Cela peut permettre à l'utilisateur de remplir rapidement ces deux coupelles avec des mélanges dont les caractéristiques sont différentes. Cela peut faciliter les essais de matières colorées, et/ou permettre de préparer plusieurs mélanges de couleurs et matité/brillance différentes destinées au maquillage de zones respectives du visage. Les coupelles peuvent être associées à des identifiants rappelant à l'utilisateur à quelle zone du visage un mélange contenu dans une coupelle donnée est destiné.

Les coupelles peuvent être mobiles relativement au distributeur, étant par exemple portées par un support mobile, tel qu'une tourelle mobile en rotation par rapport au distributeur, ou par un tiroir mobile en translation par rapport au distributeur.

Le système de distribution peut comporter un couvercle de fermeture de la coupelle. Ce couvercle de fermeture est de préférence transparent pour permettre à l'utilisateur de visualiser l'aspect du mélange contenu à l'intérieur.

Lorsque la coupelle est séparable du distributeur, elle peut être introduite le cas échéant dans un boîtier permettant de la transporter plus facilement, ce boîtier pouvant comporter le cas échéant un miroir et/ou un applicateur. Le couvercle du boîtier peut dans ce cas servir de couvercle pour la coupelle.

Le volume de la coupelle peut être compris entre 2 et 1000 mm³, mieux entre 100 et 1000 mm³, encore mieux entre 250 et 750 mm³.

Le ou les produits de base délivrés dans la coupelle sont de préférence des fonds de teint, mais en variante il s'agit de produits de maquillage des lèvres ou des paupières.

La coupelle est de préférence de forme symétrique de révolution. En variante, elle est de contour polygonal ou autre. Son plus grand diamètre intérieur, ou celui du cercle inscrit dans le cas d'un contour non circulaire, est de préférence compris entre 2 et 100 mm, préférentiellement entre 5 et 40 mm. Sa profondeur est de préférence comprise entre 1 et 10 mm, mieux entre 3 et 8 mm. Sa profondeur est de préférence comprise entre 1 et 10 mm. De préférence, la taille et la forme de la coupelle permettent soit une application directe du mélange sur la peau, soit une préhension au doigt ou avec un applicateur. La coupelle peut être réalisée avec un matériau élastiquement déformable, ce qui permet par exemple d'inverser la concavité du fond de la coupelle et de vider celle-ci plus facilement ou de l'utiliser pour appliquer le produit.

La coupelle peut être dépourvue de mélangeur ; dans ce cas, les produits de base peuvent arriver du distributeur dans la coupelle à l'état non mélangé, par des orifices de distribution respectifs distincts. En variante, le distributeur intègre un mélangeur et les produits de base arrivent déjà mélangés dans la coupelle.

La coupelle peut aussi intégrer un mélangeur statique, comme détaillé plus loin, qui est alimenté par des orifices de remplissage distincts du distributeur, et qui délivre de préférence le mélange dans une cavité de la coupelle située au-dessus du mélangeur.

L'invention a encore pour objet un procédé de préparation d'un produit de maquillage, comportant l'étape consistant à remplir une coupelle d'un système de distribution tel que défini plus haut avec au moins un produit de base provenant du distributeur.

Les produits présenteront des effets de matité/brillance différents

Plusieurs produits peuvent être délivrés dans le fond de la coupelle, puis mélangés à l'aide du doigt ou d'un applicateur, ou d'un mélangeur statique intégré à la coupelle.

La coupelle est de préférence remplie par le dessous. Il a été proposé dans le passé des systèmes de distribution à l'aide d'une sonotrode.

La coupelle selon l'invention n'est pas destinée à entrer en vibration pour distribuer le ou les produits amenés par le ou les canaux d'alimentation de celle-ci. Elle est différente d'une sonotrode. De préférence, la coupelle est réalisée en matière plastique.

### Mélangeur intégré à l'interface de sortie

Il existe un intérêt pour bénéficier d'un système de distribution capable de délivrer un mélange pouvant être facilement utilisé, notamment prélevé par l'utilisateur, sans nécessiter une action additionnelle de mélange de la part de celui-ci.

L'invention a encore pour objet un système de distribution comportant un distributeur ayant des canaux de sortie de produits de base et une interface de sortie séparable du distributeur, cette interface ayant un mélangeur statique, délivrant de préférence le mélange dans une cavité où il peut être prélevé.

Le mélangeur statique peut être situé sous la cavité précitée. Le système de distribution est alors particulièrement adapté à la création de compacts, en utilisant comme interfaces de sortie des coupelles à mélangeur statique intégré. Dans ce cas, on vient remplir la cavité de la coupelle en produit par le dessous. Après passage dans le mélangeur statique, les produits de base mélangés viennent recouvrir le mélangeur.

Il est possible d'utiliser plusieurs interfaces de sortie et de les remplir avec des mélanges respectifs différents, sans avoir à purger le mélangeur, ce qui réduit les pertes de produit. L'interface de sortie peut être à usage unique, le cas échéant.

De préférence, le mélangeur statique comporte une chambre centrale communiquant avec des canaux d'admission des produits de base. Cette chambre centrale peut communiquer avec une chambre périphérique comportant une série de cloisons qui agissent comme des déflecteurs pour le mélange, et créent un cisaillement de celui-ci.

La chambre périphérique peut comporter une cloison annulaire ajourée définissant des ajours par lesquels passe le mélange en circulant dans la chambre périphérique. Les chambres centrale et périphérique peuvent être fermées supérieurement par une paroi qui définit le fond de la cavité recevant le mélange.

Le fond de la chambre périphérique peut être de forme hélicoïdale autour de l'axe de la coupelle et de hauteur diminuant au fur et à mesure que l'on progresse vers la sortie. Cette dernière peut déboucher en avant d'une rampe de liaison entre le fond de la chambre périphérique et la paroi supérieure du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le mélangeur peut comporter un corps extérieur dans lequel est reçue une pièce formant le coeur du mélangeur, le corps extérieur fermant radialement à l'extérieur la chambre périphérique et comportant un montant qui sépare les chambres centrale et périphérique.

Le corps extérieur du mélangeur et le coeur du mélangeur peuvent être chacun réalisés d'une seule pièce par moulage par injection.

### Volume mort réduit

Il existe un intérêt pour réduire les pertes de produit lors des changements de formulation du mélange, ainsi que pour permettre de faire varier le plus rapidement possible le produit du mélange au cours de l'application, notamment en cas de couplage du distributeur avec un aérographe.

Le produit de base peut quitter la cartouche par un canal de sortie de la cartouche, ce canal de sortie débouchant à l'extérieur du distributeur ou à proximité de sa surface externe.

Le canal de sortie peut notamment déboucher dans une zone de prélèvement du mélange ou à proximité de celle-ci, notamment à moins de 5 mm, mieux à moins de 3 mm, mieux à moins de 1mm, mieux à fleur.

mm². La section transversale du canal de sortie est par exemple comprise entre 1 et3

Ainsi, chaque produit de base provenant d'une cartouche peut quitter le distributeur sans se mélanger avec un produit de base d'une autre cartouche et l'on minimise le volume mort qui ne peut être prélevé et susceptible d'accroître l'inertie du système. Le produit est plus rapidement disponible sans avoir à circuler dans des canaux spécifiques du boîtier du distributeur, évitant une étape fastidieuse de purge si changement de cartouche.

L'extérieur du distributeur peut être la zone de prélèvement du produit, notamment lorsque le distributeur est réalisé avec une coupelle non prévue pour être enlevée, dans laquelle le mélange est distribué, ou une zone du distributeur destinée au montage d'une interface de sortie amovible, qui définit la zone de prélèvement. Cette interface de sortie peut comporter une coupelle telle que définie ci-dessus. Cette zone de montage correspond par exemple à l'extrémité du boîtier du distributeur en l'absence de l'interface de sortie. La zone de montage peut être sensiblement plane et perpendiculaire à l'axe longitudinal du boîtier du distributeur.

Le distributeur peut comporter trois cartouches de produits de base.

Le distributeur peut comporter des logements pour recevoir les cartouches, lesquelles sont de préférence reçues de façon amovible dans le distributeur. Ce dernier peut comporter des passages pour des conduits des cartouches définissant les canaux de sortie.

La longueur de ces conduits est de préférence telle que les conduits se situent légèrement en retrait du fond ou à affleurement de la cavité servant au prélèvement du produit, ou en variante légèrement en retrait ou à affleurement de la face d'extrémité du boîtier du distributeur définissant la zone de montage.

Ces conduits des cartouches peuvent être des embouts servant à entraîner en déplacement des pistons au sein des cartouches, comme détaillé plus haut.

### Interfaces de sortie multiples

Il existe un besoin pour pouvoir, à l'aide d'un même système de distribution, réaliser facilement des maquillages différents, et pouvoir si on le souhaite maquiller des zones aussi différentes que la peau, les lèvres ou les cils ou sourcils.

Le système de distribution peut comporter un ensemble comportant un distributeur d'au moins un produit cosmétique, notamment de maquillage, et au moins deux interfaces de sortie pouvant se monter chacune de façon amovible sur le distributeur, ces interfaces de sortie permettant de recevoir le ou les produits délivrés par le distributeur, étant de préférence choisies parmi les suivantes :
- interface de sortie comportant un contenant, notamment une coupelle permettant un prélèvement du produit au doigt ou à l'aide d'un applicateur,
- interface de sortie permettant de délivrer le produit à un système de pulvérisation, notamment un aérographe,
- interface de sortie comportant plusieurs régions de réception du produit, mobile par rapport au distributeur,
- interface de sortie permettant de délivrer le produit à un embout de distribution.

De préférence, l'ensemble comporte au moins trois desdites interfaces de sortie, mieux les quatre interfaces de sortie.

Le distributeur peut comporter au moins deux produits de base différents et permettre de délivrer ceux-ci dans des proportions variables, et de préférence, le distributeur comporte trois produits de base différents et permet de délivrer ceux-ci dans des proportions variables.

Chaque interface de sortie peut comporter une base permettant sa fixation sur le distributeur. Cette fixation peut s'effectuer à l'aide de vis par exemple, mais de préférence la base est agencée pour permettre un démontage et un remplacement d'une interface de sortie sans nécessité d'outil. Il s'agit par exemple d'une fixation quart de tour ou à l'aide d'une bague de verrouillage externe.

L'interface de sortie et/ou le boîtier du distributeur peuvent comporter des joints d'étanchéité permettant une communication étanche entre le boîtier du distributeur et l'interface de sortie. Le cas échéant, le distributeur est agencé pour reconnaître l'interface de sortie qui est montée dessus, par exemple grâce à identifiant de l'interface de sortie sous forme de reliefs spécifiques, lesquels sont détectés par le distributeur, ou d'une puce électronique reconnue par le distributeur. Cela peut permettre d'adapter le fonctionnement du distributeur à l'interface de sortie montée dessus. Le distributeur peut communiquer à un système informatique des informations sur l'interface de sortie qu'il porte, et le système informatique peut afficher en fonction de ces informations un écran spécifique et/ou lancer un programme spécifique de commande des paramètres de fonctionnement du distributeur, afin par exemple d'adapter la dose distribuée et/ou le débit à la nature de l'interface de sortie.

Plusieurs interfaces de sortie peuvent être proposées initialement à l'utilisateur avec un distributeur commun au sein d'un même emballage, par exemple un coffret ou une boîte de carton.

L'invention a encore pour objet un procédé de maquillage, comportant l'étape consistant à sélectionner une interface de sortie, à la monter sur le distributeur, et à délivrer le ou les produits contenus dans le distributeur dans celle-ci.

### Cartographie et apprentissage

Le terme « cartographie » s'entend ici comme un procédé d'indexation d'une matité/brillance et d'une zone, avec enregistrement.

Il se peut qu'une personne nécessite plusieurs niveaux de matité/brillance avec une seule couleur et/ou couvrance pour l'ensemble des zones.

La cartographie peut concerner des applications sur des zones plus petites qu'un cm2. Toutefois, l'oeil nu a alors du mal à discerner si le résultat obtenu est adéquat, et il est préférable de substituer l'évaluation à l'oeil nu par une évaluation instrumentée, avec grossissement. L'application de petites quantités de matière colorée peut être faite au doigt, avec des outils classiques tels que des pinceaux, ou avec des applicateurs spécialisés.

La cartographie peut être générée lors d'une période d'apprentissage, pendant laquelle l'utilisateur effectue des tests avec des mélanges sur différentes zones du visage ; une fois renseignée, la cartographie peut ensuite être utilisée pour le maquillage de tous les jours.

Des interfaces graphiques spécifiques peuvent servir pendant la période d'apprentissage et pendant la période d'utilisation de la cartographie.

En particulier, le système de distribution peut être utilisé avec une interface graphique où l'opérateur voit le visage, qui est par exemple schématisé, figuratif ou précis comme une photographie ou une simulation 3D. Dans ce cas, l'opérateur peut pointer une partie du visage sur l'écran pour voir apparaître et/ou délivrer la matité/brillance adéquate.

L'interface graphique peut aussi laisser apparaître les autres zones du visage où l'utilisation de la même matité/brillance est adéquate.

Pour réaliser la cartographie, l'opérateur applique une couleur (avec une matité/brillance donnée) puis réalise l'évaluation.

Les zones du visage peuvent être traitées les unes après les autres ; par exemple, on réalise l'exercice sur une partie de la joue, puis sur le nez, etc....

Une autre possibilité consiste à réaliser un mélange de matité/brillance donnée, et appliquer sur plusieurs zones ce même mélange. L'opérateur doit alors chercher la zone du visage pour laquelle la matité/brillance est adaptée. Le mélange est ensuite indexé dans le système informatique en l'attribuant à la ou aux zones du visage pour lesquelles il convient.

Selon l'un de ses aspects, indépendamment ou en combinaison avec les autres aspects de celle-ci, et notamment ce qui précède, l'invention a pour objet un procédé d'apprentissage d'un système de distribution comportant un distributeur permettant de distribuer un mélange de matité/brillance variable, et un système informatique permettant de sélectionner une matité/brillance et de mémoriser des données, comportant les étapes consistant à :
a) Sélectionner au moins une matité/brillance à l'aide d'une interface du système informatique,
b) délivrer à l'aide du distributeur au moins un mélange de la matité/brillance sélectionnée,
c) évaluer le ou les mélanges distribués après application sur au moins une zone du visage,
d) mémoriser les caractéristiques d'au moins un mélange, notamment un mélange que l'utilisateur souhaite pouvoir rappeler, et d'au moins une zone sur laquelle il a été testé.

Cette mémorisation peut s'effectuer notamment en vue d'une distribution ultérieure de ce mélange pour maquiller ladite zone.

De préférence, le système informatique est agencé pour permettre à l'utilisateur de signaler si le résultat du test est satisfaisant ou non, voire de le renseigner sur la comparaison à un test effectué précédemment.

On peut aussi réaliser un mélange de matité/brillance donnée, et chercher la zone du visage pour laquelle il est adapté. On enregistre alors le mélange en l'attribuant à la ou aux zones du visage pour lesquelles il est adapté, dans une table de correspondance qui servira par la suite, pour, partant d'une zone de la peau, en déduire le mélange à utiliser.

On peut procéder de la même façon avec d'autres mélanges pour réaliser une cartographie de l'ensemble du visage et ainsi avoir une table de correspondance complète du visage.

On peut aussi réaliser un mélange donné, l'appliquer sur une zone donnée, puis faire varier le mélange jusqu'à obtenir le mélange le plus adapté. On enregistre alors le mélange en l'attribuant à la ou aux zones du visage pour lesquelles il convient, dans une table de correspondance qui servira par la suite, pour, partant d'une zone de la peau, en déduire le mélange à utiliser.

De préférence, le système informatique évalue et mémorise les quantités utilisées zone par zone. Un tel procédé, par « touches d'essai », permet d'identifier le ou les produits dont a besoin la personne qui veut se maquiller. Ainsi, le système de distribution peut servir sur les points de vente pour conseiller les personnes qui veulent se maquiller ou à la maison pour bien définir les produits à commander.

L'interface du système informatique comporte de préférence un écran tactile affichant la matité/brillance du mélange lors de sa sélection.

L'interface peut afficher un visage et permettre de renseigner le système informatique en sélectionnant la zone sur le visage affiché.

Le système informatique est de préférence agencé pour permettre d'associer une zone, des paramètres de reconstitution du mélange, et la date de l'essai et/ou tout autre identifiant du mélange.

Le système informatique est de préférence également agencé pour permettre d'associer en outre à ladite zone, aux paramètres de reconstitution du mélange, et à la date ou à l'identifiant du mélange, au moins l'une des données suivantes : le nom de la zone, la période de l'année, le nom d'un évènement, un identifiant de l'utilisateur, l'âge de l'utilisateur.

Les étapes a) à c) peuvent être renouvelées au moins une fois avant la mémorisation des caractéristiques du mélange à l'étape d).

Le système informatique peut être agencé pour rechercher dans une base de données une référence de produit commerciale, sur la base des caractéristiques du mélange identifié comme convenant à au moins une zone donnée, et la communiquer à l'utilisateur.

La sélection à l'étape a) peut s'effectuer avec l'aide d'un système expert externe ou non au système informatique.

Le système expert peut analyser une image de l'utilisateur, pour proposer une matité/brillance de mélange au moins sur la base de l'image analysée.

L'étape a) peut être précédée par la proposition d'une matité/brillance et d'une zone à tester avec un mélange de cette matité/brillance, par le système informatique à l'utilisateur.

Le système informatique peut être agencé pour permettre à l'utilisateur de le renseigner sur l'appréciation du résultat du test de l'étape c) et pour générer une proposition de modification du mélange à sélectionner lors du retour à l'étape a).

Le système informatique peut être agencé pour proposer au moins une matité/brillance de mélange à l'étape a) en fonction d'une zone d'application renseignée par l'utilisateur.

Le système informatique peut être agencé pour proposer au moins une zone d'application à l'étape a), en fonction d'une matité/brillance renseignée par l'utilisateur.

Le distributeur peut délivrer à l'étape b) au moins deux mélanges de matité/brillance différentes, de préférence séparés, en vue de leur application simultanée sur la zone de test.

Cela peut faire gagner du temps, et faciliter la comparaison des résultats.

L'invention a encore pour objet un procédé de maquillage à l'aide d'un système de distribution selon cet aspect de l'invention, dans lequel :
a) L'utilisateur adresse une requête concernant un besoin de maquillage au système informatique,
b) celui-ci génère en retour une proposition de matité/brillance en vue du maquillage d'une zone associée, sur la base de l'apprentissage effectué préalablement, et
c) le système informatique pilote le distributeur pour la production du mélange de la matité/brillance proposée, notamment si celle-ci est validée par l'utilisateur.

Un tel procédé peut utiliser une cartographie précédemment établie avec l'utilisateur.

L'invention a encore pour objet un produit programme d'ordinateur comportant des instructions de code permettant, lorsque exécutées dans un système informatique, d'amener le système informatique à :
- Permettre à l'utilisateur de sélectionner au moins une matité/brillance et/ou une zone d'application, notamment à l'aide d'une interface telle qu'un écran tactile,
- piloter un distributeur de façon à délivrer un mélange de la matité/brillance sélectionnée par l'utilisateur,
- permettre à l'utilisateur de déclencher la mémorisation de la matité/brillance du mélange et d'une zone d'application associée, notamment en vue d'une distribution ultérieure du même mélange, notamment sur la même zone.

Le produit programme d'ordinateur peut comporter des instructions de code permettant, lorsque exécutées dans un système informatique, d'amener le système informatique à :
- Recevoir une requête de l'utilisateur concernant un besoin de maquillage, notamment à l'aide d'une interface telle qu'un écran tactile,
- proposer sur la base au moins de données générées par le procédé d'apprentissage tel que défini ci-dessus, au moins une matité/brillance et/ou une zone d'application,
- piloter un distributeur pour la production du mélange de la matité/brillance proposée, notamment si celle-ci est validée par l'utilisateur.

La personne peut intégrer dans la cartographie le niveau de matité/brillance qu'elle désire pour chaque zone du visage, zone par zone ou ensemble de zones par ensemble de zones. Le système interprétera alors les mélanges qu'il doit réaliser pour assurer les couleurs, couvrance désirées et la matité/brillance voulu.

La cartographie peut être modifiée par la suite. Ainsi, il est possible de réaliser la cartographie sur le plan des matité/brillances dans un premier temps, puis sophistiquer la cartographie en définissant par la suite les niveaux de couleur et couvrance.

### Aide à distance

Il est souhaitable de pouvoir aider l'utilisateur à se maquiller, notamment à choisir les bonnes matité/brillance.

L'invention, selon l'un de ses aspects, indépendamment ou en combinaison avec ses autres aspects, notamment ce qui précède, a ainsi pour objet un procédé de maquillage comportant les étapes consistant à :
- permettre l'établissement d'une liaison vidéo, par exemple via internet, entre une caméra d'un premier site et un deuxième site,
- permettre au deuxième site de piloter directement ou indirectement un distributeur présent sur le premier site, ce distributeur permettant de faire varier matité/brillance d'un mélange distribué,
- permettre à une personne présente au premier site d'appliquer le mélange distribué et d'envoyer vers le deuxième site une image correspondante, pour recevoir en retour une information relative au résultat de maquillage.

Le deuxième site peut notamment comporter un écran de visualisation qui permet à un conseiller présent devant cet écran de voir le résultat du maquillage avec le produit distribué par le distributeur et de conseiller la personne qui s'est maquillée. Ce conseiller peut agir en retour sur le distributeur pour modifier la matité/brillance du mélange et l'adapter au mieux au visage de la personne présente sur le premier site. Ainsi, cette personne gouverne le mélange délivré par le distributeur. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

Le cas échéant, l'acquisition vidéo peut être calibrée à l'aide d'une mire, ou avec le mélange distribué par le distributeur sur une surface de référence. Cela permet ensuite une visualisation plus fidèle du maquillage effectué sur le premier site.

De préférence, la liaison vidéo entre les deux sites est une liaison duplex.

Le premier site peut recevoir un tutoriel du deuxième site, le cas échéant.

Des identifiants des produits de base peuvent être communiqués au deuxième site ; cela peut permettre de connaître précisément la couleur de chacun des produits de base.

Le procédé peut comporter la mémorisation des paramètres de réglage du distributeur, une fois qu'un mélange donné est estimé satisfaisant. De préférence, cette mémorisation peut être commandée depuis le deuxième site. La mémorisation peut avoir lieu dans le système informatique présent sur le premier site et/ou sur un serveur externe.

Une variante peut consister à faire travailler une personne pour aider le maquillage de plusieurs personnes. Ce mode de mise en oeuvre permet le développement des « Make-Up Artists » et leur travail soit au sein d'un institut, soit via internet. Cela permet aussi le maquillage de personnes aux moyens limités, telles que les personnes malvoyantes, ou discernant mal les couleurs, ou âgées, ou manquant de confiance en elles.

### Pilotage par interface tactile

Il existe un besoin pour faciliter le pilotage du système de distribution et notamment le choix de la matité/brillance du mélange distribué.

Selon un autre de ses aspects, indépendamment ou en combinaison avec ses autres aspects, et notamment avec ce qui précède, l'invention a ainsi pour objet un système de distribution comportant un distributeur et un système informatique de pilotage du distributeur, ce système informatique comportant un écran tactile sur lequel peut être affichée la matité/brillance du mélange et un moyen de sélection déplaçable sur l'écran, pour faire varier la matité/brillance du mélange distribué.

De préférence, l'écran affiche des matité/brillances extrêmes entre lesquelles la matité/brillance du mélange peut être sélectionnée, en déplaçant le moyen de sélection entre ces matité/brillances extrêmes.

L'écran peut afficher une échelle de matité/brillances entre au moins deux matité/brillances, ou une surface, notamment de contour triangulaire, au sein de laquelle le moyen de sélection peut se déplacer. Cette surface peut localement faire apparaître la matité/brillance du mélange en fonction par exemple de la distance à chacun des sommets, matérialisant chacun un produit de base pur.

Le système informatique peut effectuer une partie des calculs nécessaires pour déterminer les fractions de chacun des produits de base conduisant à un mélange de la matité/brillance recherchée.

Le système informatique peut être un téléphone intelligent, un photophone, une tablette, un ordinateur individuel. En variante, il est intégré au boîtier du distributeur.

Le système informatique peut disposer d'une caméra. Cette dernière peut être utilisée notamment pour l'acquisition d'une image de l'utilisateur et/ou du mélange.

Le système informatique peut être agencé pour afficher une image d'un visage, afin de faciliter le repérage des zones sur lesquelles le mélange doit être appliqué.

### Couplage du système de distribution à un moyen de pulvérisation

Le système de distribution peut comporter ou être connecté à un moyen de pulvérisation du mélange, de préférence un aérographe.

L'invention vise, selon un autre de ses aspects, à perfectionner encore les systèmes de distribution comportant un système de pulvérisation, de préférence un aérographe, et a pour objet, selon l'un de ses aspects, indépendamment ou en combinaison avec les autres aspects, et notamment ce qui précède, un ensemble comportant :
- Un moyen de pulvérisation, de préférence un aérographe comportant une chambre de prélèvement soumise à un courant d'air d'entraînement,
- un distributeur comportant au moins deux compartiments contenant des produits de base différents, les produits étant délivrés au moyen de pulvérisation de préférence par des orifices de distribution distincts.

Le distributeur peut comporter trois cartouches contenant des produits de maquillage de matité/brillances différentes.

L'aérographe peut comporter un stylet définissant la chambre de prélèvement, le stylet se fixant sur le distributeur ou sur une interface de sortie fixée au distributeur, ou faisant partie intégrante de cette interface de sortie.

Le système de distribution peut comporter un circuit de contrôle du fonctionnement du distributeur, permettant de faire varier la proportion des produits de base délivrés dans la chambre de prélèvement, au cours du fonctionnement de l'aérographe. Les proportions peuvent être modifiées en fonction du déplacement de l'aérographe relativement à la surface sur laquelle le mélange est pulvérisé. Ce déplacement peut être mécanisé, le cas échéant.

Ce circuit de contrôle peut comporter ou être constitué par un système informatique tel que défini plus haut.

Le boîtier du distributeur peut servir de poignée lors de la manipulation d l'ensemble pour délivrer le mélange.

Le distributeur peut comporter une caméra et/ou un ou plusieurs capteurs tels que des accéléromètres afin de localiser automatiquement la zone sur laquelle le mélange est appliquée, et pouvoir régler automatiquement la matité/brillance en fonction de la position, le cas échéant.

L'invention a encore pour objet un procédé de maquillage à l'aide d'un ensemble tel que défini ci-dessus, dans lequel un mélange est pulvérisé sur la peau à l'aide du moyen de pulvérisation, notamment de l' aérographe.

La composition du mélange peut être modifiée au cours du déplacement de l' aérographe relativement à la peau. Un dégradé peut être réalisé.

Cet aspect de l'invention repose sur la constatation que le distributeur peut être utilisé pour alimenter le système de pulvérisation, notamment l'aérographe, tout en permettant au système de distribution d'être suffisamment réactif pour autoriser un changement de la matité/brillance du mélange distribué en cours de maquillage du visage, notamment lorsque la zone à maquiller change.

Il peut être avantageux que la distribution des produits se fasse de façon itérative, notamment avec des temps de distribution non déphasés entre les différents produits.

Cela peut rendre plus facile de faire varier la composition du mélange distribué au cours du temps.

Le mélange peut être réalisé directement dans l'aérographe, sans pratiquement aucun volume mort gênant, permettant ainsi de changer le mélange pulvérisé en temps réel.

La dépression créée dans la chambre de prélèvement est suffisante pour entraîner les produits de base, sans pour autant gêner le dosage.

La dépression qui règne dans la chambre de prélèvement est par exemple comprise entre 10 mbar et 200 mbar, mieux entre 50 et 150 mbar, encore mieux entre 75 et 125 mbar.

La viscosité des produits de base mesurée à 1 atm et à 25°C avec un viscosimètre rotatif CONTRAVES TV équipé d'un mobile MS-r3 ou MS-r4 à la fréquence de 60 Hz, après 10 minutes de rotation du mobile, est par exemple comprise entre 0,05 Pa.S et 50 Pa.S

La section des canaux d'arrivée des produits de base dans la chambre est par exemple comprise entre 1 et 3 mm², mieux entre 2 et 3 mm².

L'alimentation en produit s'effectue de préférence de façon continue.

Il est encore possible d'appliquer simultanément des produits de base non miscibles ou réactifs, comme un gel aqueux et un gel huileux, qui vont se déposer de façon pixellisée directement sur la peau, produisant une sorte de gel/gel in situ, des silicones réactives, ou des produits de coloration réagissant ensemble. Les ratios en produits de base peuvent être ajustés en fonction du résultat particulier souhaité. Par exemple, dans le cas des gels aqueux et gels huileux, on pourra faire varier le ratio correspondant au volume du premier produit de base sur le volume du deuxième produit de base entre 10/1 et 1/10, mieux entre 5/1 et 1/5.

### Système de localisation ou d'auto-localisation

Le système de distribution selon l'invention peut disposer d'un système de localisation ou d'auto-localisation.

On appelle système de localisation, un moyen de saisir, de la part de la personne, la zone qu'elle va traiter. Ceci peut être réalisé notamment par des systèmes laissant libre au moins une main. Ainsi, on peut utiliser une interface d'un système informatique telle qu'un écran tactile, un joystick, ou un système de reconnaissance vocale.

On appelle système d'auto-localisation, un moyen de saisir, sans que la personne n'intervienne, la zone à traiter. Ceci peut être réalisé par un ou plusieurs accéléromètres qui déduisent, par le fait des mouvements, les directions que la personne vise ou par une caméra et un système de reconnaissance d'image.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 est une vue schématique en perspective d'un exemple de système de distribution conforme à l'invention,
- la figure 2 est une vue arrière du système de distribution de la figure 1,
- la figure 3 illustre le prélèvement de produit délivré par le système de distribution,
- la figure 4 est une vue schématique en perspective, avec enlèvement de certains composants, du système de distribution de la figure 1,
- la figure 5 représente isolément et partiellement une cartouche de produit de base pour le distributeur,
- la figure 6 représente le haut de la cartouche avec la pièce d'entraînement,
- la figure 7 représente isolément la pièce d'entraînement, en perspective,
- la figure 8 représente isolément un support de la cartouche,
- la figure 9 représente le mécanisme d'entraînement du distributeur,
- la figure 10 représente l'un des moteurs isolément, accouplé au reste du mécanisme d'entraînement,
- la figure 11 représente une carte électronique de commande des moteurs,
- la figure 12 est une coupe transversale du distributeur,
- la figure 13 représente le boîtier du distributeur sans l'interface de sortie,
- la figure 14 représente isolément un premier exemple d'interface de sortie, en vue de dessus,
- les figures 15, 15A, 16, 16A et 17 représentent d'autres exemples d'interfaces de sortie,
- les figures 18 et 19 sont deux autres vues de l'interface de sortie de la figure 17,
- la figure 20 représente isolément le mélangeur statique,
- la figure 21 est une coupe axiale de l'interface de sortie de la figure 17,
- la figure 22 représente une autre interface de sortie, destinée à coopérer avec un aérographe,
- la figure 23 représente en transparence les différents canaux de l'interface de sortie de la figure 22,
- la figure 24 illustre l'interface de sortie des figures 22 et 23 reliée à un aérographe,
- les figures 25, 27 à 29, 29A, 29B et 29C représentent d'autres exemples d'interfaces de sortie,
- la figure 30 illustre le pilotage du distributeur à l'aide d'un terminal portable,
- la figure 31 représente un exemple d'interface graphique permettant de piloter le distributeur,
- la figure 32 représente un autre exemple d'interface graphique,
- la figure 33 illustre un exemple d'évolution de l'interface graphique de la figure 32 au cours de l'utilisation du dispositif,
- la figure 34 représente un autre exemple d'interface graphique,
- les figures 35 et 36 représentent d'autres exemples d'interfaces graphiques,
- la figure 37 illustre l'évolution de l'interface de la figure 36 au cours de l'utilisation du dispositif,
- la figure 38 représente une interface graphique d'un exemple de système informatique selon l'invention,
- la figure 39 représente un exemple de table de correspondance,
- la figure 40 est un schéma en blocs illustrant des étapes d'un exemple de procédé selon l'invention,
- les figures 41 à 44, et- 46 sont des vues analogues à la figure 40 d'autres exemples de procédés,
- la figure 45 représente un exemple de support permettant l'application de plusieurs compositions colorées différentes,
- la figure 47 illustre un système permettant l'échange d'informations avec un conseiller distant, et
- la figure 48 illustre un support comportant une pluralité de logements contenant des mélanges différents.

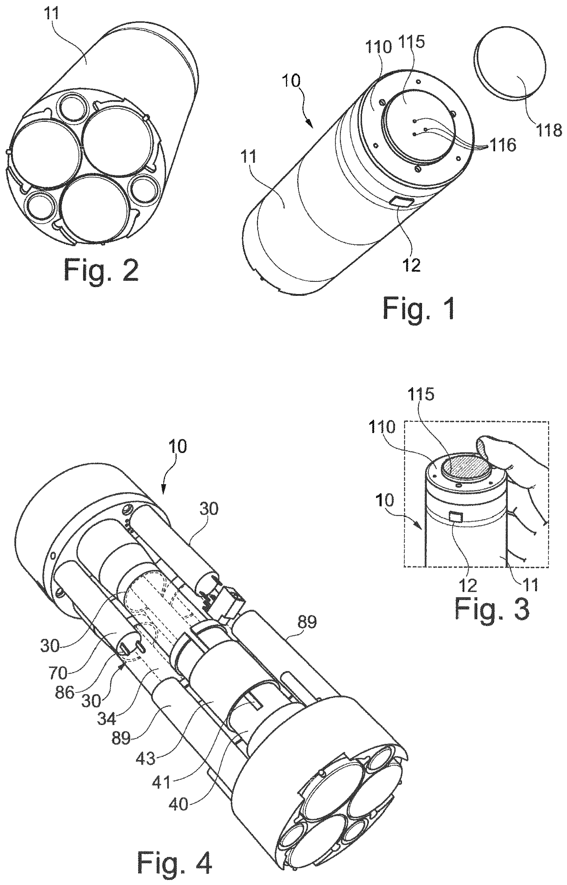

Un exemple de système de distribution 10 pouvant convenir à l'invention est représenté sur les figures 1 et 2, comporte un distributeur 11 qui est muni en partie supérieure d'une interface de sortie 110 par laquelle s'effectue la distribution d'un produit cosmétique de formulation personnalisée.

Le distributeur 11 peut être manipulé d'une main. Sa longueur, hors interface de sortie, est par exemple comprise entre 140 et 160 mm et son diamètre entre 40 et 60 mm.

Le système de distribution 10 peut comporter, comme illustré, un moyen d'actionnement permettant de contrôler la distribution, par exemple un bouton-poussoir 12.

Lorsque l'utilisateur appuie sur le bouton-poussoir 12, le distributeur 11 délivre le produit à partir d'informations qui lui ont été préalablement communiquées par un système informatique, par exemple à l'aide d'une transmission sans fil, comme cela sera détaillé plus loin. Le fonctionnement du bouton-poussoir 12 peut être programmé depuis une interface du système informatique, de façon à délivrer en continu le mélange tant qu'une pression est exercée, ou délivrer uniquement une dose prédéfinie, indépendamment de la durée pendant laquelle l'utilisateur appuie sur le bouton-poussoir.

Comme visible notamment sur la figure 4, le distributeur 11 loge plusieurs cartouches 30 contenant chacune un produit de base, le distributeur 11 permettant de doser la quantité de chacun des produits de base qui est distribuée de façon à obtenir après un mélange des doses distribuées un produit présentant les propriétés recherchées.

Chacune des cartouches 30 peut être introduite dans le boîtier du distributeur 11 par l'arrière, comme illustré à la figure 2. Dans l'exemple considéré, le distributeur 11 reçoit trois cartouches 30, mais l'invention s'étend également au cas où le nombre de cartouches 30 est différent.

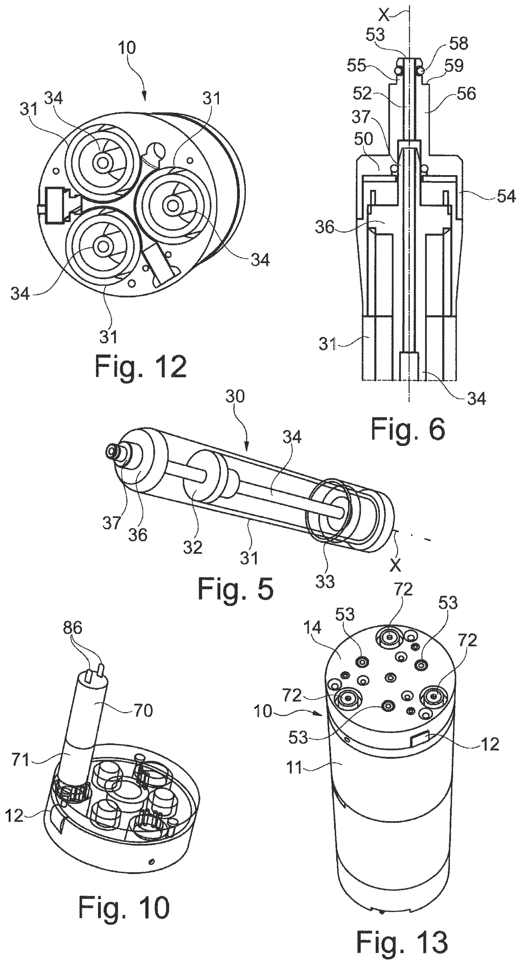

On a représenté isolément à la figure 5 une cartouche 30. Celle-ci comporte un corps 31 dans lequel peut se déplacer selon l'axe longitudinal X de la cartouche un piston 32 de façon à diminuer le volume d'un réservoir 33 situé sous le piston 32, contenant le produit de base correspondant. Le volume du réservoir est de préférence compris entre 2 et 5 mL, étant par exemple de l'ordre de 3 mL.

Le piston 32 est entraîné en déplacement selon l'axe X par une tige creuse 34, filetée extérieurement, venant en prise avec un filetage correspondant traversant le piston 32.

La tige 34 définit un canal par lequel le produit contenu dans le réservoir 33 peut circuler lorsque le piston 32 se déplace dans le corps 31 dans le sens d'une diminution du volume du réservoir 33.

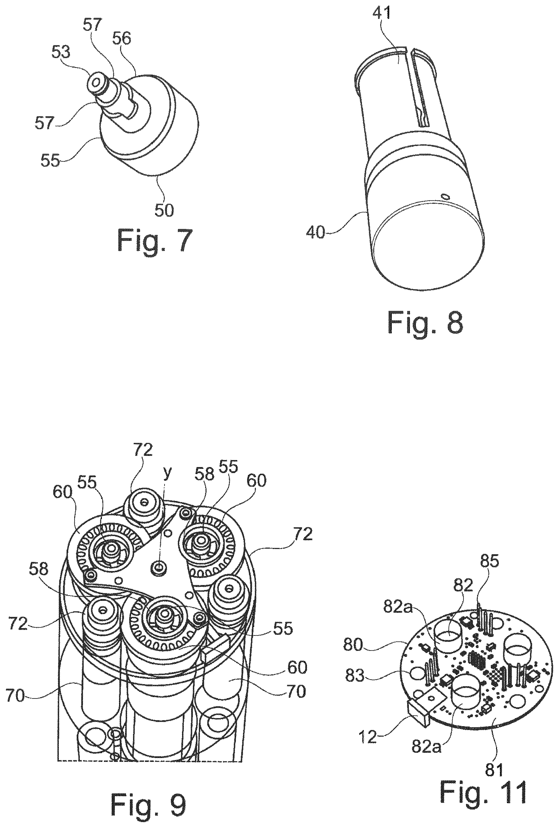

La tige 34 est entraînée en rotation autour de l'axe X par une tête 36 qui peut tourner relativement au corps 31, et communique avec une canule 37. Chaque cartouche 30 est montée dans le distributeur 11 avec une pièce de support 40, que l'on a représentée isolément à la figure 8, qui comporte un manchon de serrage 41 fendu axialement, sur lequel peut coulisser une bague de verrouillage 43, visible sur la figure 4.

Lors de la mise en place d'une cartouche 30, la pièce de support 40 est engagée sur celle-ci, du côté opposé à la canule 37, et la bague de verrouillage 43 déplacée sur le manchon 41 pour serrer la pièce de support 40 sur le corps 31. La pièce de support 40 permet d'immobiliser la cartouche 30 dans le boîtier du distributeur 11.

La tête 36 de la cartouche 30, présentant la canule 37, est coiffée par une pièce d'entraînement 50, représentée isolément à la figure 7, qui vient serrer la tête 36 de façon à pouvoir tourner autour de l'axe X avec elle.

Lorsque la pièce d'entraînement 50 est entraînée en rotation autour de l'axe X, sa rotation est transmise à la tête 36, qui peut tourner relativement au corps 31 et entraîner avec elle en rotation la tige 34.

La force de friction du piston 30 sur la surface intérieure du corps 31 est suffisante pour que le piston 32 ne tourne pas relativement au corps 31, de sorte que la rotation relative de la tige 34 et du piston 32 provoque le déplacement de celui-ci selon l'axe X. Ce déplacement s'accompagne d'une diminution du volume du réservoir 33 et d'une remontée du produit de base contenu dans la cartouche 30 par la tige 34, puis dans la canule 37.

La pièce d'entraînement 50 comporte un canal interne 52 alimenté par la canule 37 et qui débouche à l'extérieur par un orifice de distribution 53. Ce canal 52 est formé par un embout 36. La pièce d'entraînement 50 présente une jupe de montage 54 qui recouvre axialement la tête 36 de la cartouche 30. Cette jupe de montage 54 se raccorde par une paroi transversale 55 à l'embout 56.

L'embout 56 comporte des reliefs 57 permettant son accouplement en rotation avec une roue dentée 60, visible notamment sur la figure 9, appartenant à un mécanisme d'entraînement du boîtier du distributeur 11.

Dans l'exemple considéré, les reliefs 57 se présentent sous la forme de deux ergots diamétralement opposés, formant saillie sur l'embout 56 à sa base, qui s'engagent dans des encoches correspondantes de la roue dentée 60.

L'embout 56 présente une partie rétrécie qui comporte une gorge accueillant un joint torique 58. La partie rétrécie se raccorde par un épaulement 59 au reste de l'embout.

La tête 36 de la cartouche 30 peut porter un joint torique qui assure un couplage étanche entre la canule 37 et la pièce d'entraînement 50.

Le mécanisme d'entraînement comporte des moteurs électriques 70, équipés de réducteurs 71, visibles notamment à la figure 10. L'arbre de sortie de ces réducteurs est accouplé à une roue menante 72 qui engrène avec la roue dentée 60.

Dans l'exemple considéré, les axes X longitudinaux de chacune des cartouches sont disposés à 120° les uns par rapport aux autres, autour de l'axe longitudinal Y du boîtier du distributeur 11.

Les moteurs 70 sont disposés entre les cartouches 30, les axes de rotation des moteurs étant également disposés à 120° les uns des autres autour de l'axe Y du distributeur 11. De cette façon, on obtient une construction compacte du distributeur 11.

Les motoréducteurs présentent avantageusement un couple supérieur à
70nN.m. Par exemple, on utilise un moteur de référence Maxon 118392 associé au réducteur planétaire Maxon 218418. Un tel moteur est de diamètre 10 mm, de puissance 1.5 W, de tension nominale 3 V, de vitesse de ralenti 1300 tours/min et de couple maximum 1.5 mNm. Le réducteur est de diamètre 10 mm, de réduction absolue 256 / 1 et de couple 0,2 Nm.

Un circuit électronique 81, représenté isolément à la figure 11, est présent dans le voisinage de l'extrémité supérieure du boîtier du distributeur 1. Ce circuit électronique 81 comporte une carte 80 qui est traversée par des passages 83 pour les axes de sortie des réducteurs 71, ainsi que des ouvertures 82 pour les parties rétrécies 55 des embouts d'entraînement 56.

Des manchons 82a peuvent être fixés sur la carte 80 pour faire barrière vis-à-vis d'éventuelles fuites de produit vers le carte 80. Les embouts 56 traversent les manchons 82a, de préférence avec un faible jeu.

La carte 80 porte le bouton-poussoir 12 précité et supporte un certain nombre de broches de sortie 86 servant à l'alimentation des moteurs 70.

Le circuit électronique 81 comporte un microcontrôleur ou analogue permettant de piloter les différents moteurs 70, afin de distribuer la quantité désirée de chacun des produits de base. La résolution de délivrance des produits de base est par exemple comprise entre 0,001 et 0,003 mL, étant par exemple de l'ordre de 0.0025 mL.

Le boîtier du distributeur 11 loge également une batterie dont les accumulateurs 89, se situent avantageusement comme on le voit sur la figure 4, chacun dans le prolongement d'un moteur 70.

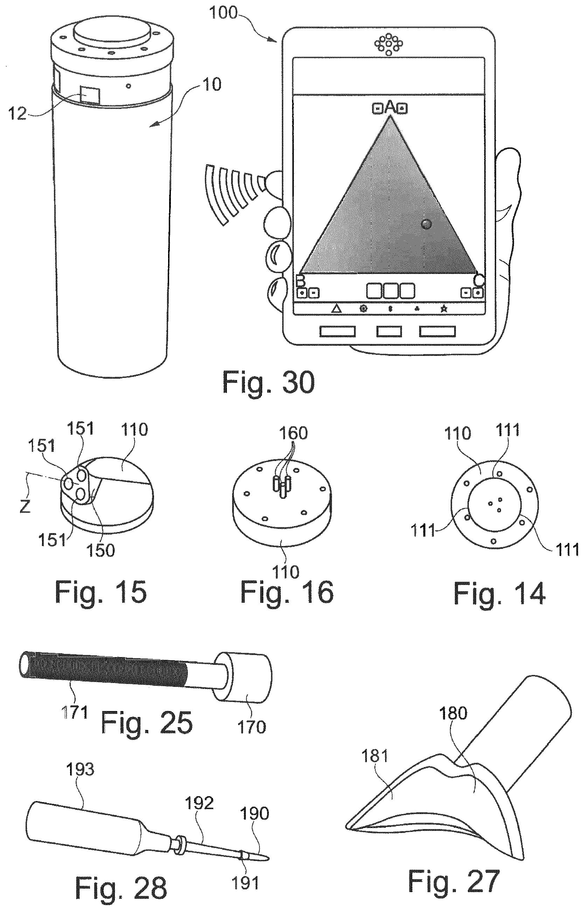

De préférence, comme illustré à la figure 30, le pilotage du distributeur 11 s'effectue à l'aide d'un système informatique 100 tel qu'un terminal portable, par exemple un téléphone intelligent, une tablette par exemple de marque « Ipad » ou un ordinateur personnel.

La transmission des informations de pilotage du distributeur 11 par le terminal 100 s'effectue de préférence sans fil, par exemple par une liaison Bluetooth.

Dans un exemple particulier, la carte électronique 81 permet de gérer les points suivants :
- calcul du volume de chaque produit à délivrer en fonction de la consigne de fraction volumique de chaque produit, du mode de fonctionnement (continu, dose ou purge), de la valeur du débit ou du volume,
- mesure des courants d'alimentation des moteurs 70,
- communication Bluetooth avec le système informatique 100,
- gestion du bouton 12 de délivrance des produits,
- gestion de l'interrupteur marche/arrêt,
- gestion de l'affichage de la ou des leds,
- charge de la batterie.

La carte 80 comporte par exemple les composants suivant :
- microcontrôleur CC2541 de Texas Instrument,
- led CMS bleue pour donner des informations de statut à l'utilisateur,
- fusible thermique de protection,
- quartz à 32 MHz,
- interrupteur marche/arrêt

Le microcontrôleur Texas Instrument CC2541 intègre une mémoire flash programmable de 256 ko de RAM ainsi que de nombreuses fonctionnalités. Ce microcontrôleur peut être programmé en C, dans l'environnement IAR Embedded Workbench.

Les orifices de sortie 53 des cartouches 30 débouchent sensiblement à l'extrémité supérieure du boîtier du distributeur 11, comme on peut le voir sur la figure 13 notamment. La face supérieure 14 du boîtier du distributeur 11 définit une surface d'accueil pour le montage d'une interface de sortie qui canalise les produits provenant des cartouches vers une zone de prélèvement ou de distribution.

Dans l'exemple de la figure 1, cette interface de sortie 110 se présente sous la forme d'une pièce rapportée, que l'on a représentée isolément à la figure 14, qui présente à sa périphérie comme illustré des passages 111 pour des vis servant à sa fixation sur le boîtier du distributeur 11.

L'interface de sortie 110 définit dans cet exemple une coupelle 115 dans le fond de laquelle débouchent des orifices d'alimentation 116, en communication chacun par un canal interne à l'interface de sortie 110 avec un orifice de sortie respectif 53.

Ainsi, dans l'exemple considéré, les produits de base contenus dans les cartouches 30 peuvent être distribués jusqu'à la coupelle 115 sans se mélanger.

Lorsqu'il utilise le distributeur 11, l'utilisateur peut remplir la coupelle 115 avec des proportions prédéfinies de chacun des produits de base, comme illustré à la figure 3, puis peut prélever le produit présent dans la coupelle 115 en vue de l'appliquer. Ce prélèvement peut s'effectuer par exemple au doigt, comme illustré, ou à l'aide de tout applicateur cosmétique adapté. La coupelle 115 est de préférence peu profonde, ce qui facilite son nettoyage, et de diamètre suffisamment grand pour ne pas gêner l'accès au produit. La profondeur *p* de la coupelle 115 est ainsi, de préférence, comprise entre 1 et 5 mm et son diamètre *d* ou celui du cercle circonscrit lorsque le contour du creuset n'est pas circulaire, est de préférence compris entre 20 et 50 mm. On a de préférence 4 *>*=*d*/*p* <=50.

Les orifices d'alimentation 116 sont de préférence de diamètre inférieur à 3mm par exemple de l'ordre de 1 mm.

L'interface de sortie 110 peut recevoir un couvercle de fermeture 118 de la coupelle 115, pour éviter que le produit ne sèche ou soit exposé à des salissures en l'absence d'utilisation. Ce couvercle 118 est de préférence réalisé en matière plastique transparente et peut se fixer par friction, vissage ou encliquetage sur le montant de la coupelle 115, ou plus généralement à tout endroit adapté sur l'interface de sortie 110.

La contenance maximale de la coupelle 115 est de préférence comprise entre 0.02 et 0.25 ml.

De préférence, le volume défini par le volume des canaux internes de l'interface de sortie 110 entre l'entrée dans ceux-ci depuis les orifices d'alimentation 53 jusqu'aux orifices d'alimentation 116 est inférieur ou égal à 0,4ml.

Le boîtier du distributeur 11, dans sa configuration illustrée à la figure 13, c'est à dire sans l'interface de sortie 110 décrite ci-dessus, présente l'avantage de pouvoir être accouplé à diverses formes d'autres interfaces de sortie, en fonction du maquillage que l'on souhaite réaliser et/ou de la zone à traiter.

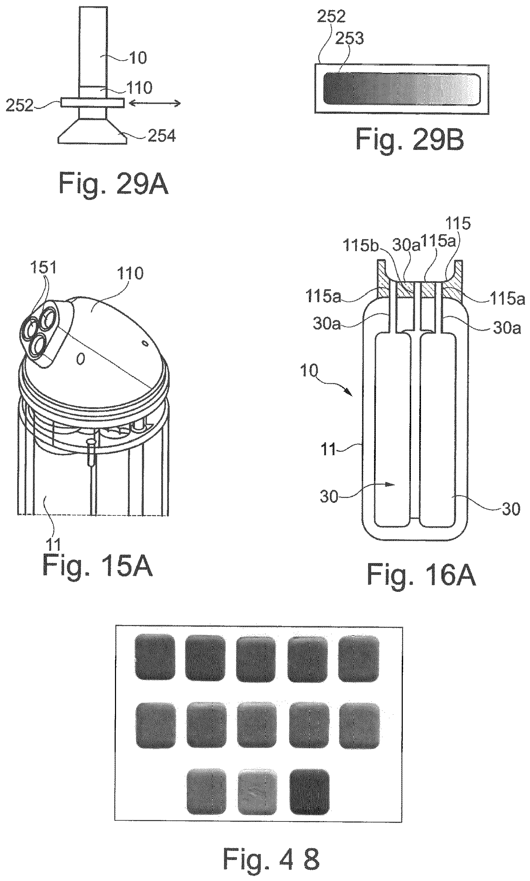

Ainsi, on a représenté sur la figure 15 une variante d'interface de sortie 110 comportant un embout de distribution 150, orienté généralement selon un axe Z qui s'étend obliquement par rapport à l'axe longitudinal Y du distributeur 11. Trois canaux internes communiquent respectivement avec les orifices de sortie 53 des différentes cartouches 30 et débouchent à l'extrémité de l'embout 150. L'interface de sortie 110 peut se monter à une extrémité du boîtier du distributeur 11, comme illustré sur la figure 15A.

Dans la variante de la figure 16, l'interface de sortie 110 comporte trois canules 160 qui communiquent respectivement avec les orifices de sortie 53. Ces canules 160 sont regroupées au centre de l'interface de sortie 110, ce qui permet le montage sur celles-ci par exemple d'un embout 170 d'application du produit sur les cils, tel qu'illustré à la figure 25, d'un embout poreux 180 pour l'application sur les lèvres, tel qu'illustré à la figure 27, ou d'un embout 190 floqué tel qu'une pointe feutre, comme illustré à la figure 28.

Dans le cas de l'embout 170 de la figure 25, celui-ci comporte par exemple, comme illustré, des stries 171 transversales, entre lesquelles débouchent des orifices d'alimentation en produit. Le mélange des différents produits de base peut avoir lieu à l'intérieur de l'embout 170, grâce à un mélangeur statique intégré, par exemple.

L'embout 180 comporte par exemple une partie en mousse à cellules ouvertes, ayant la forme des lèvres. Le mélange des produits de base peut se faire au sein d'un conduit interne de l'embout 180.

L'embout 190 peut comporter un organe d'application poreux 191, à l'extrémité d'une tige 192, se raccordant à une base 193 servant au montage sur le reste de l'interface de sortie de la figure 16.

On a illustré à la figure 16A, de façon schématique, la possibilité d'avoir comme interface de sortie 110 une coupelle 115 ayant des passages 115a pour des conduits de sortie 30a des cartouches, y compris lorsque ces conduits servent à l'entrainement en rotation de tiges filetées de déplacement des pistons. La longueur des conduits 30a est telle que ceux-ci débouchent dans le fond 115b de la coupelle ou à proximité de son fond, sans faire saillie dans celui-ci.

De préférence, la section interne des conduits 30a est faible, pour minimiser le volume mort.

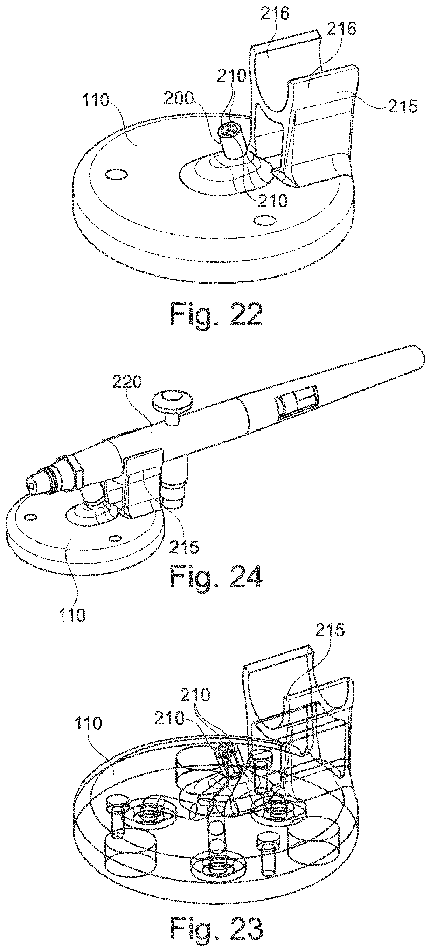

Dans l'exemple des figures 22 à 24, l'interface de sortie 110 comporte un embout 200 qui est orienté généralement obliquement par rapport à l'axe longitudinal Y du distributeur. Cet embout 200 est parcouru par des canaux internes 210, communiquant respectivement avec les orifices de sortie 53 des produits de base provenant des cartouches 30.

L'interface de sortie 110 comporte une partie de montage 215 qui permet la fixation sur l'interface de sortie 110 d'un aérographe 220, comme illustré à la figure 24.

L'embout 200 se fixe à la place du réservoir habituel de l'aérographe et les canaux 210 débouchent dans la tuyère de l'aérographe où ils sont soumis à la dépression créée par la vitesse du flux d'air d'entraînement.

Une pince est formée par deux montants 216, pour recevoir le corps du stylet de l'aérographe 220, et assurer son maintien par encliquetage.

De préférence, l'orientation de l'embout 200 est telle qu'elle permet à l'axe de pulvérisation d'être orienté sensiblement perpendiculairement à l'axe longitudinal du boîtier du distributeur 11. On peut alors se servir de celui-ci comme d'une poignée pour manipuler l'aérographe.

Les orifices de sortie 210 sont avantageusement très rapprochés, étant séparés par de fines cloisons internes de l'embout 200.

De préférence, la section de chacun des orifices de sortie est inférieure ou égale à 3 mm² sur une longueur d'au moins 5 mm.

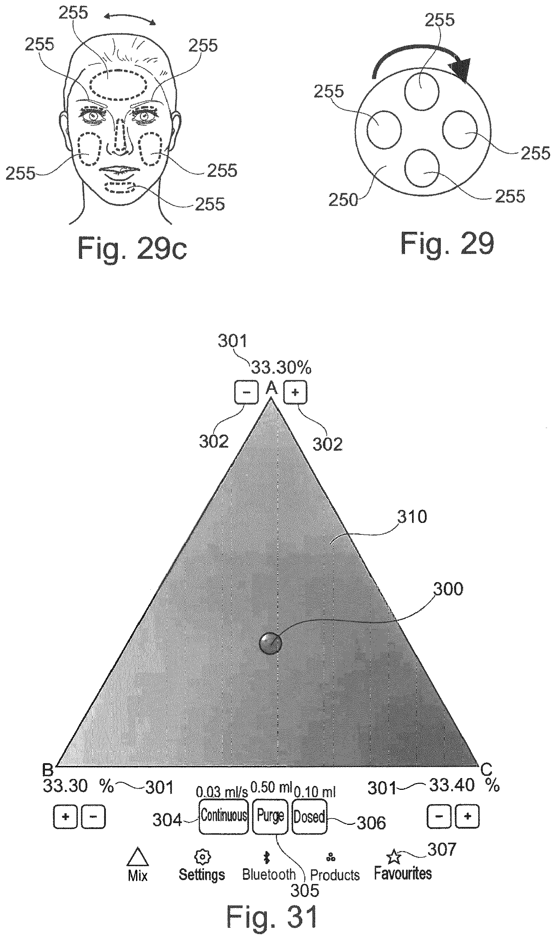

On peut encore munir le boîtier du distributeur 11 en partie supérieure d'un support mobile par rapport au boîtier, par exemple sous forme de tourelle 250, tel qu'illustré à la figure 29.

Cette tourelle 250 tourne par exemple autour d'un axe de rotation qui est confondu avec l'axe longitudinal Y du distributeur.

La tourelle 250 peut comporter plusieurs logements 255 pouvant accueillir chacun les produits délivrés par le distributeur 11 dans une position de remplissage correspondante. Pour remplir successivement les différents logements, on fait effectuer à la tourelle 250 une rotation, par exemple d'un quart de tour, à chaque fois. La présence de plusieurs logements 255 peut permettre de distribuer des produits de formulations différentes, à partir des différents produits de base, de façon par exemple à faire varier la teinte des produits présents dans les différents logements 255.

On a illustré à la figure 29C un support ayant des logements disposés sur celui-ci sensiblement comme les différentes zones d'un visage ; chaque logement peut contenir un mélange dont la matité/brillance est adaptée à la partie correspondante du visage. Ainsi, il est facile pour l'utilisateur de savoir où appliquer le mélange prélevé dans un logement donné.

On peut utiliser le distributeur 11 pour délivrer un mélange dont la formulation change au cours du temps et recueillir le mélange dans un contenant mobile par rapport au distributeur, de telle sorte que le mélange soit déposé en un emplacement du contenant qui varie dans le temps, afin de réaliser un dégradé.

Par exemple, comme illustré aux figures 29A et 29B, le système de distribution comporte une interface de sortie 110 comportant une partie fixe relativement au distributeur et une partie mobile 252 présentant un logement 253 pour recevoir le mélange.

Par exemple, le distributeur 11 est disposé dans ce cas avec les orifices de sortie des cartouches vers le bas, et équipé d'un mélangeur, de telle sorte que le mélange tombe sous l'effet de son poids dans le logement 253. Un moteur peut déplacer la partie mobile de l'interface de sortie relativement au distributeur, de façon synchronisée avec la variation des caractéristiques du mélange, de telle sorte que l'on obtienne un dégradé le long du logement 253, comme illustré à la figure 29B.

Le système de distribution peut comporter un socle 254 qui maintient le distributeur tête en bas.

L'interface de sortie 110, notamment lorsqu'elle comporte une coupelle, peut comporter un mélangeur statique qui assure le mélange des produits de base.

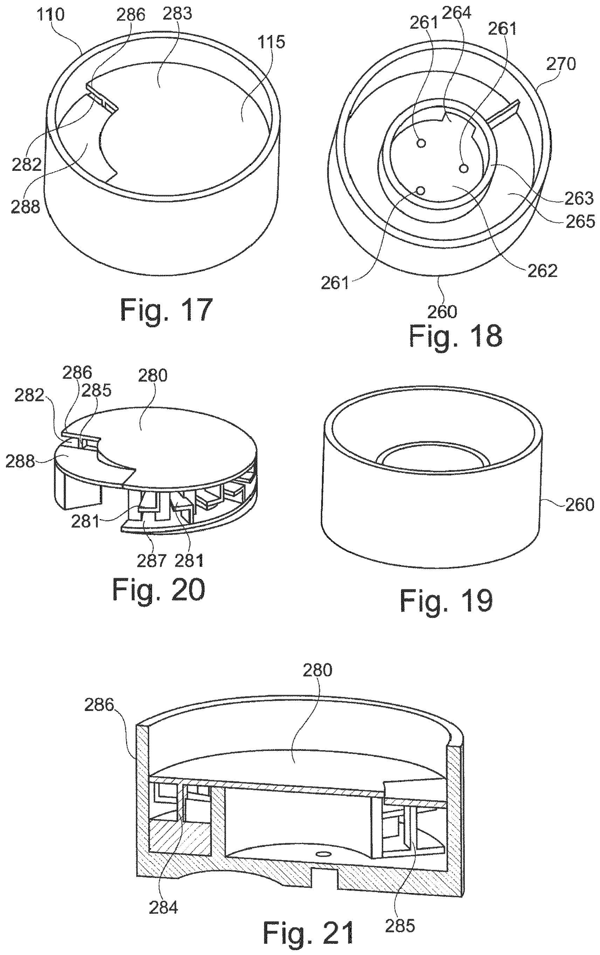

On a représenté sur les figures 17 à 21 une interface de sortie 110 comportant un tel mélangeur statique.

Cette interface de sortie 110 peut comporter un corps extérieur 260 qui se fixe sur le boîtier du distributeur 11 et qui présente un montant tubulaire extérieur 270.

Le corps 260 comporte des canaux 261 pour l'admission des différents produits de base. Ces canaux 261 débouchent dans une chambre centrale 262, délimitée par un montant tubulaire intérieur 263.

Ce montant 263 est traversé par une ouverture 264 qui débouche dans un espace annulaire 265, entre le montant intérieur 263 et le montant extérieur 270.

Un coeur de mélangeur statique 280, représenté isolément à la figure 20, est disposé dans cet espace 265.

La chambre centrale 262 peut communiquer avec une chambre périphérique comportant une série de cloisons formées par le coeur du mélangeur et qui agissent comme des déflecteurs pour le mélange, et créent un cisaillement de celui-ci.

La chambre périphérique peut comporter une cloison annulaire ajourée 284 définissant des ajours 285, dont l'un est visible sur la figure 21, par lesquels passe le mélange en circulant dans la chambre périphérique. Les chambres centrale et périphérique peuvent être fermées supérieurement par une paroi 286 qui définit le fond de la cavité recevant le mélange.

Le fond 287 de la chambre périphérique peut être de forme hélicoïdale et de hauteur diminuant au fur et à mesure que l'on progresse vers la sortie. Cette dernière peut déboucher en avant d'une rampe 288 de liaison entre le fond 287 de la chambre périphérique et la paroi supérieure 286 du mélangeur, cette rampe de liaison étant de préférence une portion d'hélice prolongeant l'hélice formée par la paroi de fond de la chambre périphérique.

De préférence la chambre périphérique comporte la cloison annulaire précitée et des cloisons radiales 281 qui forcent le mélange à circuler alternativement entre des régions supérieure et inférieure de la chambre périphérique et entre des régions radialement intérieure et extérieure, le mélange circulant par exemple d'une région supérieure et radialement extérieure à une région inférieure et radialement extérieure en passant à travers la cloison annulaire précitée.

Le corps 260 ferme radialement à l'extérieur la chambre périphérique.

Le corps extérieur 260 du mélangeur et le coeur 280 du mélangeur peuvent être chacun réalisés d'une seule pièce par moulage par injection.

Le produit gagne le coeur 280 du mélangeur statique par le passage 264, puis circule entre les montants 263 et 270 sur pratiquement une circonférence complète, jusqu'à la sortie 282.

Les nombreuses chicanes imposées par les cloisons 281 provoquent un mélange intime des composants introduits dans l'interface de sortie 110. Le mélange obtenu peut être prélevé par l'utilisateur dans le logement 283, au-dessus du mélangeur statique.

Comme indiqué précédemment, le système de distribution 10 selon l'invention comporte de préférence une interface homme machine qui permet à l'utilisateur de piloter facilement et de façon intuitive le distributeur 11. Cette interface peut appartenir à un système informatique 100 qui communique avec le distributeur 11.

On a représenté sur les figures 31 à 37 différents exemples d'interfaces tactiles pouvant permettre à l'utilisateur de choisir la matité/brillance du mélange résultant de la distribution dosée des différents produits de base.

Cette interface peut présenter, comme illustré à la figure 31, une zone de sélection de la matité/brillance, par exemple sous la forme d'un triangle dont les sommets correspondent aux matité/brillances de chacun des produits de base contenus dans les cartouches.

L'utilisateur peut déplacer un curseur 300, par exemple sous la forme d'une balle, relativement aux sommets A, B et C du triangle.

Plus il rapproche le curseur 300 de l'un des sommets, plus la fraction du produit de base correspondant est grande par rapport à la quantité totale des différents produits distribués.

La fraction de chaque produit par rapport à la quantité totale peut être indiquée en 301 par une valeur numérique sur l'interface.

L'interface peut permettre à l'utilisateur d'incrémenter ou de diminuer la quantité de chacun des produits, en agissant par exemple sur des boutons de commande 302, lesquels permettent un réglage précis de la quantité de chacun des produits de base.

La surface du triangle 310 peut avoir une matité/brillance qui varie localement de façon à être représentative en chaque point de la matité/brillance du mélange résultant de la pondération des différents produits de base dans des proportions correspondant aux coordonnées relatives en ce point.

L'interface peut comporter un bouton 305 permettant d'accéder à un menu spécifique de réglage du volume de produit qui est distribué pour purger le distributeur.

L'interface peut aussi avantageusement permettre un réglage du débit de produit grâce à des boutons 304 et 306 renvoyant vers un menu spécifique de réglage du débit.

Dans l'exemple considéré, l'interface offre le choix entre un mode de distribution continu, grâce au bouton 304, où les produits sont distribués tant que l'utilisateur appuie sur le bouton de commande 12.

La dose correspondante peut être transmise à l'interface et affichée.

Le bouton 306 permet de sélectionner un fonctionnement en mode dose, au cours duquel un appui même bref sur le bouton 12 déclenche la distribution d'une dose prédéfinie.

Pour faire varier le débit, le distributeur agit par exemple sur le rapport cyclique de fonctionnement des moteurs.

L'interface peut être agencée pour permettre à l'utilisateur de programmer ou de mémoriser les réglages qui ont sa préférence, grâce à un menu 307 d'accès à des favoris.

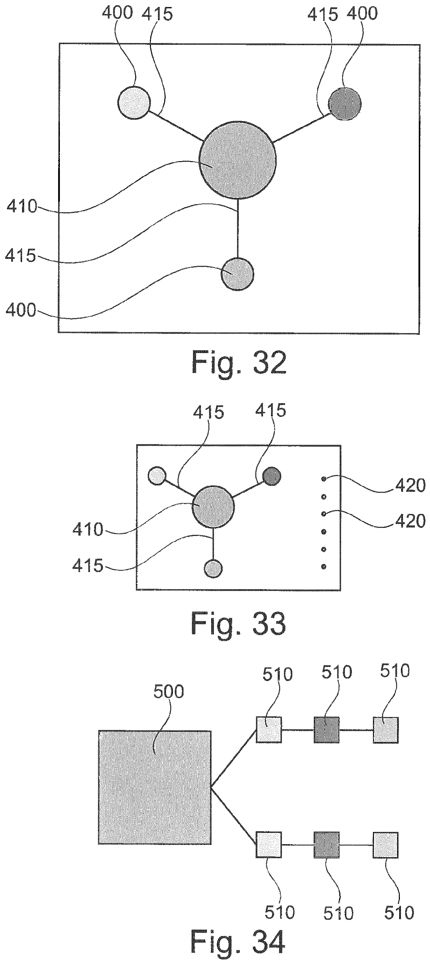

L'interface tactile illustrée à la figure 32 fait apparaître sur l'écran trois zones colorées 400, correspondant chacune à la matité/brillance de l'un des produits de base contenus dans le distributeur 10, et une zone centrale 410 faisant apparaître la matité/brillance du mélange résultant.

La quantité relative de chacun des produits de base peut être ajustée à l'aide de curseurs 415 qui se déplacent par exemple sur des lignes joignant chacune des zones 400 à la zone centrale 410.

Au cours de l'utilisation de l'interface, celle-ci peut mémoriser un réglage donné et faire apparaître sur l'écran un bouton 420 de la matité/brillance du mélange. L'utilisateur peut ensuite, en appuyant simplement sur ce bouton 420, distribuer un mélange de la matité/brillance correspondante.

Dans l'exemple de la figure 34, l'interface affiche dans une zone 500 une teinte donnée, et propose à l'utilisateur, grâce à des boutons de commande 510 chacun de la matité/brillance du produit de base correspondant, d'accroître ou de diminuer la proportion de ce produit de base dans le mélange final. La matité/brillance de la zone 500 est recalculée en fonction des actions sur les boutons de commande 510.

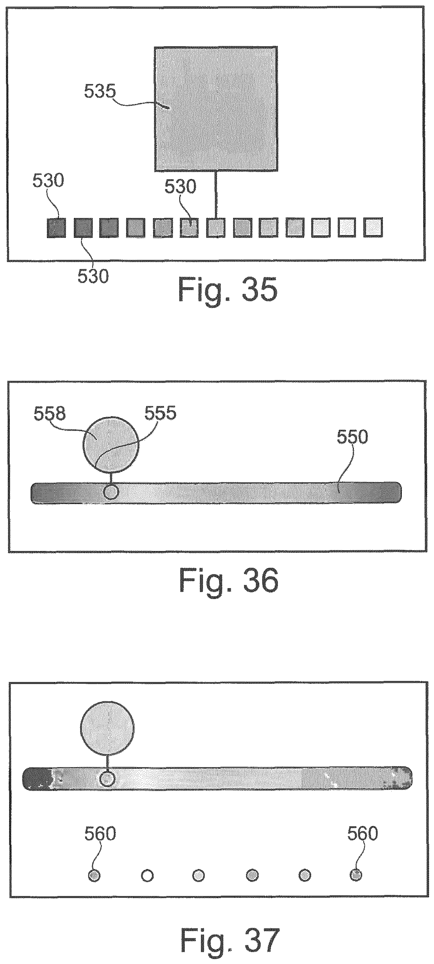

Dans la variante de la figure 35, l'interface fait apparaître un nuancier présentant plusieurs zones 530 correspondants chacune à une proportion particulière des différents produits de base.

L'utilisateur peut sélectionner l'une de ces zones, par exemple en appuyant dessus avec son doigt.

L'interface peut être agencée pour afficher à une échelle plus grande dans une zone 535 la matité/brillance sélectionnée. La programmation du distributeur 11 pour distribuer cette matité/brillance est par exemple déclenchée en appuyant sur la zone.

Dans l'exemple de la figure 36, l'utilisateur peut déplacer sur un nuancier continu 550 un curseur 555, qui fait apparaître dans une zone 558 la matité/brillance sélectionnée.

L'utilisateur peut ensuite, en appuyant par exemple dans la zone 556, déclencher l'envoi au distributeur 11 des instructions nécessaires pour que celui-ci distribue un produit de la matité/brillance sélectionnée.

On voit sur la figure 37 que l'interface peut mémoriser les différentes teintes sélectionnées, et les faire ensuite apparaître sur l'écran de façon à permettre à l'utilisateur, en appuyant sur des boutons correspondants 560, de sélectionner à nouveau très facilement une teinte précédemment choisie.

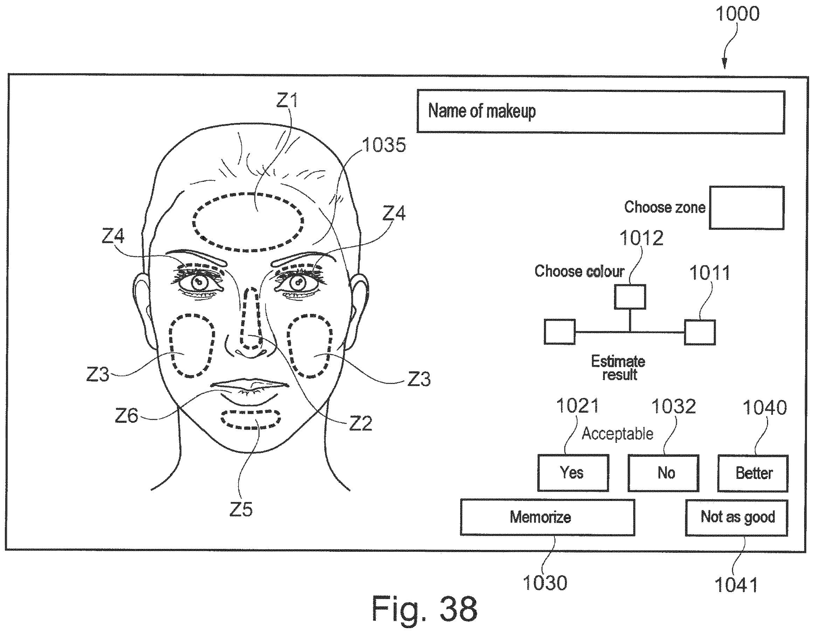

On a représenté à la figure 38 un exemple d'interface utilisateur 1000 d'un système de distribution comportant un distributeur, de préférence tel que décrit précédemment, et un système informatique 100 auquel appartient l'interface.

Le système informatique comporte ici par exemple un appareil tel qu'un ordinateur portable, une tablette ou un téléphone intelligent, fonctionnant de façon autonome ou connecté à un serveur distant.

Dans l'exemple considéré, l'interface 1000 est définie par l'écran tactile d'un tel appareil. Dans une variante non illustrée, le distributeur intègre un écran tactile ou tout autre type d'interface homme machine, et peut être utilisé sans connexion à un autre appareil.

L'appareil exécute une application, qui a par exemple été téléchargée au préalable, et qui affiche sur l'écran un visage 1035 et une série de boutons permettant à l'utilisateur d'entrer des informations.

Le visage peut comporter plusieurs zones Z1 à Z6 sélectionnables tactilement, par exemple le front, le nez, les joues, les paupières, le menton, et les lèvres.

Les boutons présents sur l'écran peuvent permettre par exemple d'entrer le nom du maquillage ou de l'utilisateur, d'afficher la zone sélectionnée, de choisir la matité/brillance, et d'informer le système informatique du résultat acceptable ou non du test effectué, voire comme illustré de donner une information concernant l'évaluation du résultat relativement à un test effectué précédemment, à savoir par exemple mieux ou « moins bien ». L'écran peut également afficher un bouton permettant de mémoriser le choix d'une matité/brillance et d'une zone, après avoir effectué un essai avec cette matité/brillance sur la zone en question.

Le choix de la matité/brillance s'effectue par exemple avec une échelle de matité/brillances similaire à celle décrite en référence à la figure 36.

Le système informatique est agencé pour mémoriser les données sous forme de table de correspondance par exemple, de manière à associer à une zone du visage des paramètres permettant de reproduire le mélange distribué lors du test. Ces paramètres comportent par exemple les teneurs relatives de chacun des produits de base du distributeur dans le mélange, la quantité Q distribuée, ainsi que des données additionnelles telles que par exemple le nom de la zone, la date de distribution du mélange et/ou tout autre identifiant du mélange, les identifiants des produits de base, la période de l'année, notamment la saison, l'âge de l'utilisateur, son sexe, son nom ou prénom, le nom d'un évènement associé au maquillage, par exemple un anniversaire, entre autres données, la quantité de produit adéquate pour la zone. Les données annexes peuvent permettre à l'utilisateur de reproduire plus facilement un maquillage qu'il a considéré comme adéquat pour une période de l'année, ou rappelant un évènement de la vie, ou pour donner un effet de rajeunissement.

Ces données peuvent être mémorisées dans le système informatique 100, par exemple dans l'appareil précité et/ou sur un serveur distant avec lequel l'appareil communique, ou encore dans une mémoire électronique intégrée au distributeur 11.

Ainsi, selon l'invention, l'utilisateur peut amener le distributeur à délivrer une première matière colorée, à l'appliquer sur une première zone du visage, puis la juger adéquate ou non. Si le résultat est satisfaisant, l'utilisateur peut l'enregistrer en l'indexant à la zone ; si le résultat est non satisfaisant, l'utilisateur peut commander une nouvelle matité/brillance pour refaire les opérations ci-dessus.

Le système informatique peut être utilisé dans ce contexte de diverses façons.

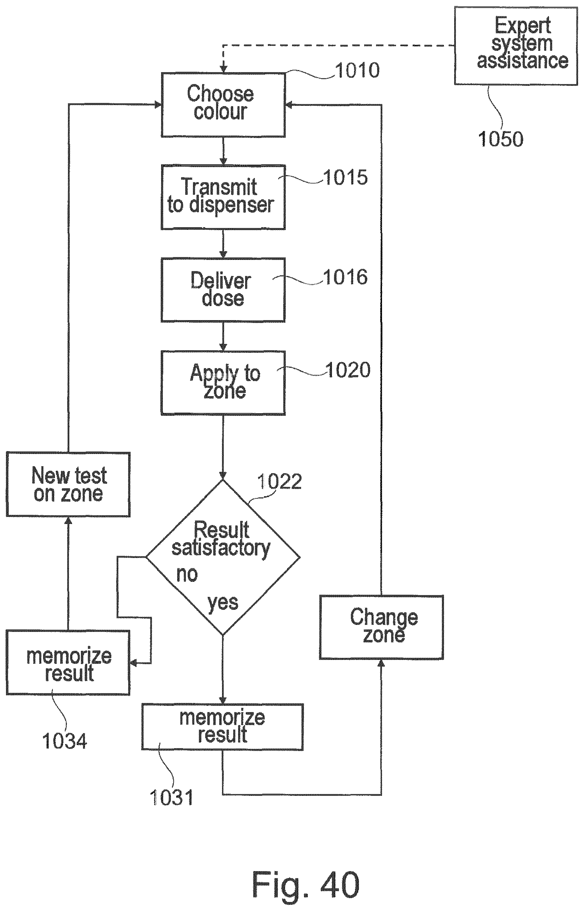

Par exemple, comme illustré à la figure 40, l'utilisateur choisi à une étape 1010 une matité/brillance à essayer, en utilisant par exemple l'échelle de matité/brillances 1011 apparaissant sur l'écran, en déplaçant le bouton de réglage 1012.

Ensuite, le choix de la matité/brillance est transmis au distributeur 11, à l'étape 1015.

Par exemple, l'appareil transmet les quantités à distribuer de chacun des produits de base, et le circuit électronique 81 se charge de commander les moteurs en conséquence.

A l'étape 1016, l'utilisateur appuie sur le bouton de commande 12 du distributeur 11, ce qui provoque par exemple la distribution d'une dose du mélange, de la matité/brillance sélectionnée par l'utilisateur.

Le mélange est par exemple distribué dans la coupelle 115, puis prélevé et appliqué par l'utilisateur sur les joues ou toute autre zone renseignée sur l'interface, à l'étape 1020.

Dans des variantes, le produit est appliqué à l'aide d'un aérographe ou par tout autre moyen tel que décrit précédemment.

L'utilisateur renseigne ensuite à l'étape 1022 le système informatique à l'aide des boutons 1021, sur le résultat.

Si l'utilisateur indique que le résultat est satisfaisant, le système lui propose par exemple de valider à l'aide d'un bouton les paramètres de l'essai afin de les mémoriser à l'étape 1031.

Si le résultat n'est pas jugé satisfaisant par l'utilisateur et signalé comme tel à l'aide du bouton 1032, le résultat peut néanmoins être automatiquement sauvegardé à l'étape 1034.

On peut ainsi indexer à chaque zone non seulement la ou les matité/brillances adéquates mais aussi la ou les matité/brillances qui ne vont pas du tout à cette zone.

L'utilisateur peut alors effectuer un nouvel essai sur la même zone en revenant à l'étape 1010.

Si l'utilisateur était satisfait du résultat, il peut également souhaiter effectuer un nouvel essai, par exemple sur une zone différente du visage.

Le cas échéant, si l'utilisateur n'est pas satisfait, l'interface peut lui proposer d'indiquer si le résultat est estimé mieux ou moins bien que l'essai précédent, à l'aide de boutons correspondants 1040 et 1041.

Dans ce cas, le système informatique peut être agencé pour déterminer si au vu des renseignements entrés par l'utilisateur une proposition peut être automatiquement effectuée quant au choix de la nouvelle matité/brillance à tester.

Le cas échéant, un questionnaire peut s'afficher pour aider le système informatique à proposer une matité/brillance au vu des essais effectués et de l'évaluation qui en a été faite par l'utilisateur ou par un professionnel qui l'assiste.

Par exemple, si la matité/brillance est jugée comme « non adéquate », le système peut recevoir de l'utilisateur une information additionnelle, par exemple « trop mat », « trop brillant » qui l'aidera à proposer une nouvelle matité/brillance plus en adéquation avec ce qu'attend l'utilisateur.

Il peut être intéressant que le système informatique puisse recevoir une information de comparaison du résultat par rapports aux essais précédents, par exemple « c'est mieux » ou « c'est moins bien » et que de là, le système soit capable d'en déduire la nouvelle matité/brillance à proposer.

Une autre option est que le système informatique puisse recevoir une information de comparaison par rapport à une cible, par exemple « c'est presqu'idéal » et que de là, le système soit capable d'ajuster automatiquement ses modifications de matité/brillance.

En l'occurrence, s'il reçoit l'information selon laquelle le résultat souhaité est presque atteint, le système peut adopter de petits niveaux de modification de la matité/brillance et revoir l'échelle de réflexion de réglage en conséquence.

Dans le cas où le système de distribution propose de lui-même les mélanges colorés à tester, il peut se fonder sur des scénarios d'essais préprogrammés et modifier le suivi du scénario selon les réussites ou échecs de l'évaluation. Ainsi, par exemple, si dès la troisième application de produit, il reçoit une information selon laquelle la matité/brillance est presqu'idéale pour l'utilisateur, le système de distribution peut sortir du programme et se laisser guider ensuite par les instructions de l'opérateur.

D'une façon générale, l'utilisateur peut être aidé par un système expert dans le choix des matité/brillances à essayer.

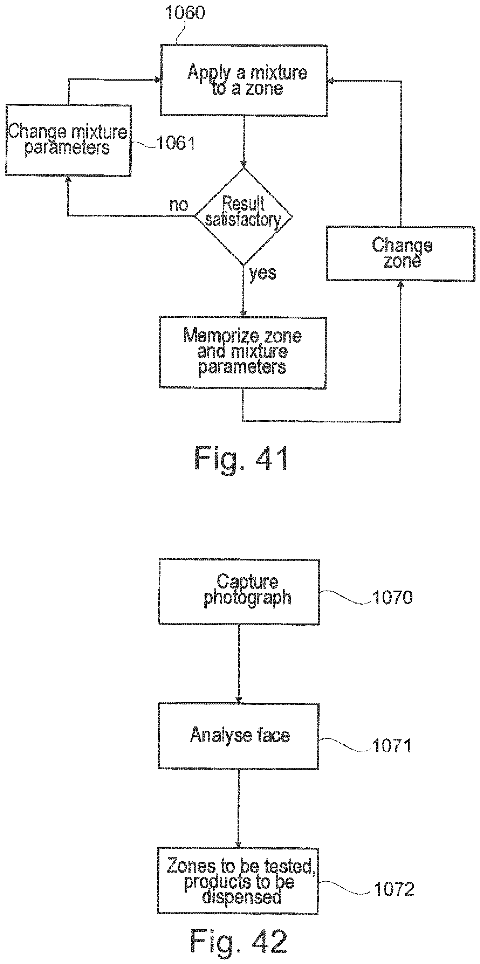

Ce système expert est par exemple un programme exécuté sur l'appareil avec lequel le distributeur communique ou sur le distributeur lui-même, qui se fonde sur des réponses à un questionnaire et/ou sur des mesures, par exemple de la matité/brillance de la peau, effectuées par un capteur spécifique ou une caméra. L'utilisateur peut ainsi se faire aider par une évaluation instrumentée, par exemple un capteur de matité/brillance. Le système expert peut encore être implémenté sur un serveur distant avec lequel l'appareil ou le distributeur échange des informations. L'opérateur peut encore envoyer une image de son visage à un spécialiste qui peut préprogrammer le choix des matité/brillances de départ. Dans un autre exemple de mise en oeuvre, l'utilisateur présente au système informatique une photo de son visage, et le système informatique est agencé pour l'analyser et créer un programme définissant les zones à tester et les premiers produits à délivrer, à la fois en matité/brillance et quantité. Par exemple, le système informatique peut être agencé pour sélectionner automatiquement des matité/brillances de maquillage à proposer à l'utilisateur en effectuant l'acquisition d'une photographie à l'étape 1070, comme illustré à la figure 42. Par exemple, l'appareil qui communique avec le distributeur 11 est équipé d'une caméra, et l'utilisateur prend une photographie de son visage. L'image est ensuite analysée à l'étape 1071, et des matité/brillances sont proposées pour chaque zone du visage à l'étape 1072, selon par exemple des règles prédéfinies d'accord des matité/brillances.

Le système de distribution peut être orienté par l'utilisateur pour décider la matité/brillance mais aussi la quantité de produit à délivrer. Par exemple, l'utilisateur peut indiquer « nez » ou « tache » et le système de distribution est agencé pour adapter la dose distribuée selon une cartographie en mémoire des doses à distribuer en fonction des zones à traiter.

Le système informatique peut guider l'utilisateur dans le choix des matité/brillances du mélange à essayer, afin de limiter le nombre d'essais nécessaires jusqu'à l'obtention d'un résultat plaisant pour l'utilisateur.

Il est ainsi possible, comme illustré à la figure 41, qu'après l'application d'un mélange délivré par le distributeur sur une zone donnée du visage à l'étape 1060, le système informatique demande à l'utilisateur si le résultat est satisfaisant ou non, et effectue de lui-même, si le résultat est considéré comme étant non satisfaisant, un changement 1061 des paramètres du distributeur de façon à modifier le mélange distribué.

L'utilisateur n'a alors qu'à effectuer un nouvel essai avec le mélange modifié.

Lorsqu'un mélange est signalé comme étant satisfaisant, le système informatique peut mémoriser les paramètres correspondants afin de permettre de recréer ultérieurement le mélange.

Le système peut alors recommencer les étapes précédentes pour une nouvelle zone d'application.

Lors des essais successifs, l'opérateur n'a pas besoin de traiter tout le visage. Il peut par exemple choisir entre 3 et 8 zones de petites surfaces, par exemple 5 zones. Le système de distribution est alors avantageusement prévu pour interpoler et/ou extrapoler les données concernant les matité/brillances considérées comme adéquates, afin de calculer les matité/brillances qui devraient être considérées comme adéquates sur des zones sur lesquelles l'exercice n'a pas été fait.

A la fin de l'apprentissage, le système peut générer une visualisation des matité/brillances adéquates sur les différentes zones, testées ou calculées.

Le système de distribution peut être agencé pour signaler si certaines matité/brillances apparaissent aberrantes, se fiant en cela à une comparaison par rapport à des cartographies types en mémoire. Ainsi, il peut proposer à l'utilisateur de refaire tout ou partie de l'exercice de cartographie.

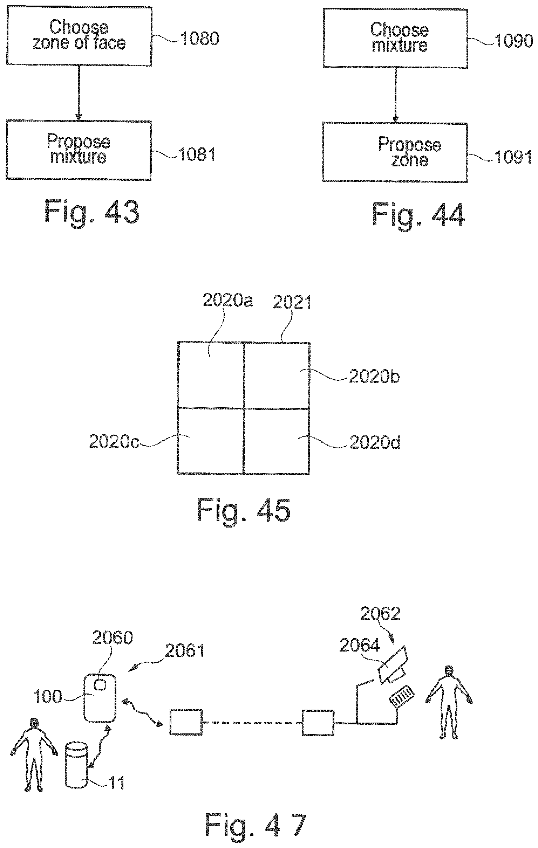

Une fois l'apprentissage du système informatique effectué, c'est-à-dire que des matité/brillances de mélanges ont été identifiées comme convenant à l'utilisateur pour maquiller certaines zones, l'utilisateur qui souhaite se maquiller n'a qu'à rappeler la zone à maquiller, à l'étape 1080 de la figure 43, et le système peut proposer automatiquement à l'utilisateur à l'étape 1081 une matité/brillance de mélange adéquate.

Dans la variante illustrée à la figure 44, l'utilisateur sélectionne une matité/brillance à l'étape 1090 et le système informatique propose à l'étape 1091 une zone où appliquer un mélange de cette matité/brillance, sur la base d'informations précédemment collectées sur la base des tests effectués.

La zone proposée est par exemple celle où une matité/brillance identique ou proche a déjà été appliquée et le résultat jugé acceptable par l'utilisateur.

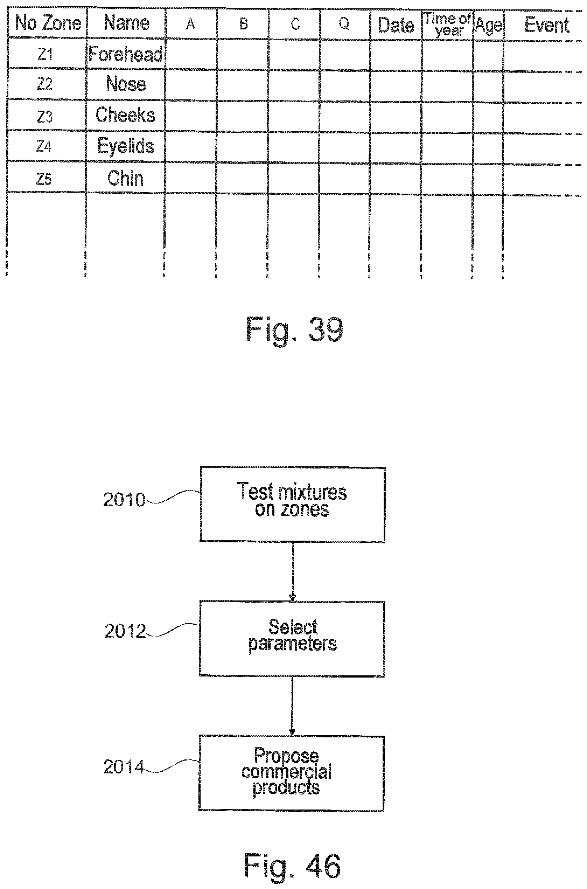

La figure 46 illustre un exemple de mise en oeuvre de l'invention où après avoir effectué des tests sur différentes zones à l'étape 2010, l'utilisateur signale au système le ou les mélanges qu'il estime procurer le meilleur résultat, ce qui permet au système de connaître les paramètres correspondants à l'étape 2012. Ensuite, à l'étape 2014, le système peut proposer à l'utilisateur des références de produits commerciaux ayant les mêmes propriétés ou des propriétés proches.

Dans une variante, le système adresse à un centre de fabrication distant les paramètres permettant de produire une composition de même formulation ou ayant les mêmes propriétés que celle du mélange que l'utilisateur a testé et trouvé satisfaisant.

La figure 45 illustre la possibilité de délivrer à l'aide du distributeur plusieurs doses 2020a à 2020d de mélanges différents, de façon juxtaposée sur un support 2021, de façon à permettre leur application sur des régions distinctes voisines d'une même zone.

L'utilisateur peut d'un seul coup appliquer une série de matité/brillances afin de cerner rapidement la matité/brillance adéquate. Les matières colorées présentes sur le support 2021 peuvent avoir été choisies par l'opérateur lui-même ou proposées par le système de distribution.

Le support 2021 est par exemple mobile par rapport au boîtier du distributeur et déplacé séquentiellement pour déposer les mélanges correspondants dans les différentes zones 2020a à 2020d, étant par exemple similaire aux supports décrits en référence aux figures 29 ou 29A. L'utilisateur peut alors facilement comparer les résultats entre les différentes régions, et signaler au système le mélange produisant le meilleur effet.

On a illustré à la figure 47 un système qui permet d'aider l'utilisateur à se maquiller, notamment à choisir les bons coloris.

Ce système permet l'établissement d'une liaison vidéo, par exemple via internet, entre une caméra 2060 d'un premier site 2061 et un deuxième site 2062.

La caméra 2060 est par exemple intégrée à une tablette ou un téléphone intelligent qui constitue le système informatique 100.

On permet au deuxième site 2062 de piloter directement ou indirectement le distributeur 11 présent sur le premier site 2061.

Ainsi, la personne présente au premier site peut appliquer le mélange distribué et envoyer vers le deuxième site 2062 une image correspondante, pour recevoir en retour une information relative au résultat de maquillage.

Le deuxième site 2062 peut comporter un écran de visualisation 2064 qui permet à un conseiller présent devant cet écran de voir le résultat du maquillage avec le mélange distribué par le distributeur et de conseiller la personne qui s'est maquillée. Ce conseiller peut agir en retour sur le distributeur 11 pour modifier la matité/brillance du mélange et l'adapter au mieux au visage de la personne présente sur le premier site. Le protocole d'échange des données entre les deux sites permet ainsi l'envoi d'instructions de commande au distributeur 11, soit directement, soit par l'intermédiaire du système informatique 100 présent sur le premier site. Ainsi, la personne présente sur le deuxième site gouverne le mélange délivré par le distributeur 11. La première personne peut se maquiller sous les yeux de la seconde. La seconde personne voit sur son écran le résultat de l'essai, et ainsi peut corriger le mélange qu'elle va commander à distance, jusqu'à obtenir le maquillage idéal.

De préférence, la liaison vidéo entre les deux sites est une liaison duplex, de sorte que l'utilisateur présent sur le premier site peut voir sur l'écran du système informatique l'image du conseiller. Ce dernier peut adresser à l'utilisateur présent sur le premier site un tutoriel, le cas échéant.

La mémorisation des paramètres de réglage du distributeur 11, une fois qu'un mélange donné est estimé satisfaisant, peut être commandée depuis le deuxième site.

Avantageusement, le système de distribution 10 est agencé pour pouvoir modifier de façon esthétique l'ensemble des matité/brillances pour chaque zone si la personne désire changer la matité/brillance de son visage. Le système de distribution peut être agencé de telle sorte qu'il suffit à l'utilisateur de modifier une seule matité/brillance dans une zone pour qu'il modifie toutes les autres. Le système de distribution peut utiliser pour cela des translations, par exemple en relevant la saturation des matité/brillances ou en décalant la teinte.

Le système de distribution peut être agencé pour recevoir la cartographie de quelqu'un d'autre, réel ou virtuel. Il peut aussi combiner la cartographie de la personne avec la cartographie d'une autre, pour sublimer le maquillage sans perdre les caractéristiques propres.

L'interface peut servir à définir des programmes de maquillage où l'on définit l'ordre des zones à maquiller ou l'ordre des matité/brillances à proposer.

### Exemples

On réalise un distributeur 11 tel que celui illustré à la figure 3 et précédemment décrit. Le distributeur est agencé pour communiquer avec une tablette 100 telle qu'un Ipad. Ce système informatique exécute une application dénommée « µMix » développée dans l'environnement spécifique d'Apple (XCode 4 et Simulateur iOS) en langage Objective C.

Elle utilise des frameworks de base Foundation, UIKit et CoreGraphics, fournissant les outils de manipulation des structures de données, des outils de calcul et les fonctionnalités liées à l'interface graphique utilisateur.

L'application utilise également le framework CoreBluetooth fournissant un accès aux périphériques Bluetoth 4 Low Energy avec les tâches principales suivantes :
- Recherche des périphériques Bluetooth 4.0 Low Energy,
- Connexion/déconnexion et gestion des paramètres de connexion,
- Communication en mode lecture et/ou écriture basée sur l'architecture GATT (Generic Attribute Profile).

L'application propose les fonctionnalités suivantes :
- Définition des fractions de produits de base,
- Choix du mode de fonctionnement lors de l'appui sur le bouton de commande12, à savoir continu, purge ou dose,
- Affichage d'un triangle de fraction volumique tel qu'illustré la figure 30 avec gestion de la fraction volumique par touché tactile sur le triangle ou par les boutons +/associés à chaque produit,
- Connexion/déconnexion Bluetooth et transfert en temps réel des consignes au distributeur,
- Réglages des débits en mode continu, et de la quantité en mode dose,
- Calcul, affichage et transfert au distributeur des fractions volumiques des produits en temps réel, en fonction de la consigne, avec la somme des fractions toujours égale à 100%,
- Récupération et affichage des couples des trois moteurs en temps réel, et
- Sauvegarde des paramètres principaux dans un fichier de configuration.

Le mode continu est un mode de distribution où le mélange des trois produits de base est distribué tant que l'utilisateur appuie sur le bouton de distribution 12. Le produit est délivré avec un débit dont une estimation est affichée au-dessus du bouton 304 « Continu ». Le choix du débit se fait dans un menu « Réglages ».

Le mode « dose » est un mode de distribution du mélange par dose, où la dose est délivrée suite à une impulsion de l'utilisateur sur le bouton de distribution 12. Une impulsion suffit et l'utilisateur peut ensuite relâcher le bouton. La dose globale de produit distribuée est celle indiquée au-dessus du bouton 306 « Dose », 0.1 ml par exemple. Ce volume peut être modifié dans le menu « Réglages ».

Le mode « purge » est un mode de distribution où une dose de mélange à fractions volumiques égales (33%) est délivrée dès que l'utilisateur a appuyé sur le bouton de distribution 12, comme dans le mode « Dose ». Une impulsion suffit et l'utilisateur peut ensuite relâcher le bouton. Quand la dose a été entièrement distribuée, on peut relâcher le bouton. Si l'on relâche avant la fin, la distribution s'arrête, même si le volume spécifié n'est pas atteint. La dose globale de produit distribuée est celle indiquée au-dessus d'un bouton 305 « Purge », 3 ml par exemple. Ce volume peut être modifié dans le menu Réglages.

L'utilisateur détermine la matité/brillance voulue avec l'application qui s'exécute sur la tablette et qui calcule les fractions des différents produits. La tablette communique cette valeur au distributeur par liaison Bluetooth.

L'électronique intégrée au distributeur 11 récupère l'information et ajuste automatiquement les débits des trois cartouches de façon à obtenir un mélange de la matité/brillance souhaitée.

Quand l'utilisateur veut utiliser le produit, il appuie sur le bouton 12 du distributeur pour faire sortir le produit. Il presse aussi longtemps qu'il veut du produit, en mode « continu ». En mode « dose », l'utilisateur presse une fois le bouton 12 et la dose prédéfinie est délivrée.

La distribution peut se faire en continu, c'est-à-dire les moteurs fonctionnent en continu, tout le volume étant distribué d'une traite, ou de façon itérative, les moteurs fonctionnant alors de façon impulsionnelle ; dans ce cas, l'intervalle de temps entre deux impulsions permet de faire varier le débit. De petits volumes sont délivrés les uns après les autres en plusieurs étapes.

Les impulsions peuvent être séparées par exemple par des intervalles de 50 ms, 100 ms ou 200 ms. La durée d'une impulsion pendant laquelle le moteur tourne va par exemple de 50 à 150 ms.

La page principale de l'application « µMix » comporte dans cet exemple les éléments suivants, comme visible notamment sur la figure 31 :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active : page principale, Réglages, Bluetooth, Produits et Favoris ;
- Bouton Continu 304 pour sélectionner le mode de distribution de produits en continu ;
- Bouton Purge 305 pour sélectionner le mode Purge ;
- Bouton Dose 306 pour sélectionner le mode de distribution par dose avec le volume de dose associé au bouton Dose ;
- Balle bleue 300 que l'utilisateur peut déplacer dans le triangle volumique en la faisant glisser ou avec un double tapotement ;
- Boutons « - » 302 pour chaque produit A, B et C : diminue la fraction de produit sélectionné en se déplaçant le long de la droite reliant le point au sommet du produit sélectionné ;
- Boutons « + » 302 pour chaque produit A, B et C : augmente la fraction de produit sélectionné en se déplaçant le long de la droite reliant le point au sommet du produit sélectionné ;
- Fraction volumique en pourcentage de chaque produit : modifiable par l'utilisateur et mise à jour en temps réel selon la consigne des boutons 302 + et - et de la position de la balle 300.

Lors de la modification des fractions volumiques par déplacement de la balle ou par les boutons + et -, les valeurs des fractions volumiques des produits A, B et C sont mises à jour automatiquement. Lorsque les fractions volumiques sont modifiées avec les boutons + et -, la balle 300 est déplacée automatiquement à la position correspondante sur le triangle.

Au démarrage de l'application exécutée sur la tablette, celle-ci se connecte automatiquement au distributeur 11 s'il est détecté. Lorsque le distributeur s'éteint ou que la connexion Bluetooth est coupée, la tablette se déconnecte. Quand l'utilisateur agit sur les curseurs de réglage des proportions des produits A et B, les valeurs sont transmises en temps réel au distributeur 11.

La page Réglage de l'application comporte les éléments suivants :
- Barre de statut en haut de l'écran : indique l'état de la connexion Bluetooth ou µMix s'il n'y a pas de connexion Bluetooth ;
- Barre d'onglets en bas de l'écran : permet de sélectionner la page active :
   page principale, Réglages, Bluetooth ou Info ;
- Partie « Volumes » avec un champ de texte à remplir par l'utilisateur pour définir le volume de la dose, en ml (2 ml par exemple) et un champ pour le volume de la purge, en ml (3 ml par exemple). Les doses minimales sont dans cet exemple de 0.023 ml et les doses maximales 9.90 ml (3 x 3.3 ml) ;
- Partie « Débit » avec la sélection du débit : rapide (» 0.03 ml/s), moyen (» 0.02 ml/s) ou lent (» 0.01 ml/s) ;
- Partie « Dose » avec le choix du mélange itératif, pour distribuer un mélange de produits avec des petits volumes délivrés les uns après les autres en plusieurs étapes ;
   Dans le cas contraire, le volume total de chaque produit est distribué d'une traite ;
- Partie « Image Triangle » pour choisir l'image du triangle qui sera affichée sur la page principale afin de pouvoir afficher un triangle avec les matité/brillances délivrées par le distributeur 11. En utilisant un bouton « Choix image » de la page « Réglages » on accède à un album.

La page « Produits » de l'application comporte dans l'exemple considéré les éléments suivants :
- Choix de la valeur de chaque produit en unités de pas codeurs, de 0 à 1414.

Chaque unité correspond à un volume de produit délivré de 2.33 µl, qui est la plus petite quantité que le distributeur dans cet exemple peut délivrer ; lorsque cette feuille est affichée, ce sont les valeurs de produits de cette feuille qui sont transmises en temps réel au distributeur. Dès que la feuille n'est plus affichée, les valeurs envoyées au distributeur sont celles de la feuille principale avec le triangle ;
- Affichage des couples moteur A, B et C en temps réel avec rafraichissement toutes les 45 valeurs.

Le mode de délivrance des produits est le mode dose directe ou itératif, selon l'option choisie dans la feuille Réglage.

La page « Favoris » permet de sauvegarder des configurations en fichier. Elle donne accès dans l'exemple considéré à 10 fichiers, à savoir « Configuration 1 » à « Configuration 10 » en plus du fichier par défaut. Ces fichiers enregistrent par exemple les paramètres suivants
- fractions des produits A, B et C
- volume de Purge
- volume de Dose,
- débit rapide, moyen ou lent,
- mode Dose, Purge ou Continu,
- distribution continue ou itérative.

### Exemple

On réalise plusieurs produits de base (les proportions sont massiques).

Les formules F1 et F2 sont riches en charges (et de couleurs différentes). La formule F3 est riche en nacre (oxychlorure de bismuth).

| | | F1 | F2 | F3 |
|---|---|---|---|---|
| | | % massique | % massique | % massique |
| A | Dimethicone copolyol vendu sous la référence KF 6017 par la société Shin Etsu | 2 | 2 | 1,8 |
| | Etsu Bis PEG/PPG-14/14 dimethicone + Cyclopentasiloxane vendu sous la référence ABIL EM 97 par la société Goldschmidt | 1 | 1 | 0,9 |
| | Cyclopentasiloxane | 17,65 | 17,65 | 15,92 |
| | Phenyl trimethicone vendu sous la référence DC556 par la société Dow Corning | 2 | 2 | 1,8 |
| | Ethyl hexyl methoxycinnamate | 3 | 3 | 2,7 |
| | Squalane | 1 | 1 | 0,9 |
| | Dispersion d'oxychlorure de bismuth dans hydroxystéarate d'éthyl (2) hexyle 70:30 (Timiron Liquid Silver^{®} de Merck) | 0 | 0 | 10 |
| B | Cyclopentasiloxane | 7 | 7 | 6,29 |
| | Oxyde de fer faune enrobé de stearoyl glutamate d'aluminium NAI-C33-9001-10 de la société Miyoshi Kasei | 1,62 | 1,1 | 1,45 |
| | Oxyde de fer rouge enrobé de stearoyl glutamate d'aluminium NAI-C33-8001-10 de la société Miyoshi Kasei | 0,29 | 0,6 | 0,26 |
| | Oxyde de fer noir enrobé de stearoyl glutamate d'aluminium NAI-C33-7001-10 de la société Miyoshi Kasei | 0,13 | 0,13 | 0,12 |
| | Dioxyde de titane (anatase) enrobé de stearoyl glutamate d'aluminium NAI-TAO-77891 de la société Miyoshi Kasei | 9,96 | 10,17 | 8,95 |
| C | Talc vendu sous la référence Micro Ace P3 par la société Nippon Talc | 0,5 | 0,5 | 0,45 |
| | Poudre de nylon 12 vendue sous la référence SP 500 par la société Toray Industries | 0,5 | 0,5 | 0,45 |
| D | Eau déminéralisée | 36,15 | 36,15 | 32,53 |
| | 1,3-Butylene glycol | 3 | 3 | 2,7 |
| | Sulfate de magnésium | 0,7 | 0,7 | 0,63 |
| | Solution de maltose hydrogénée | 0,5 | 0,5 | 0,45 |
| E | Alcool éthylique 96° dénaturé | 13 | 13 | 11,7 |
| | TOTAL | 100 | 100 | 100 |

### Mode opératoire

On pèse les constituants de la phase A dans le bêcher principal et l'on agite en maintenant à température ambiante sous Moritz (1000 tr/min). La phase B est préparée séparément en broyant trois fois à la tricylindre, le mélange de pigments et de cyclopentasiloxane. Cette phase B est ensuite ajoutée en maintenant l'agitation, ainsi que les charges (phase C).

La phase aqueuse D est aussi préparée séparément, en pesant dans un bêcher le butylène glycol, le sulfate de magnésium, la solution de maltose hydrogénée et l'eau à température ambiante. La phase aqueuse D est placée sous agitation à l'aide d'un barreau magnétique jusqu'à homogénéisation. C D E L'émulsion se fait à température ambiante : on verse la phase aqueuse D dans la phase grasse en augmentant progressivement la vitesse d'agitation (Moritz) jusqu'à 4000 tours/mn. On maintient l'agitation pendant 7 mn. Puis on ajoute enfin la phase E (alcool éthylique) pendant les 3 dernières minutes de 5 l'émulsification.

### Test

Le système a été testé avec :
- F1 dans le compartiment A
- F2 dans le compartiment B
- F3 dans le compartiment C

| | |
|---|---|
| M1 (les proportions dans le mélange distribué sont volumiques) : | A 30%, B 20%, C 50% |
| M2 : | A 30%, B 50%, C 20% |
| M3 : | A 30%, B 30%, C 40% |

M1, M2, M3 sont appliquées sur différentes zones du visage. On obtient des couleurs différentes, et des résultats de matités brillance différentes

### RESUME

lxxxv) Système de distribution d'un produit comportant un distributeur recevant au moins deux cartouches ayant chacune un réservoir et contenant respectivement des premier et deuxième produits de base, le premier produit de base comportant l'un au moins d'une huile, d'un pigment interférentiel ou à reflet métallique, le deuxième produit de base comportant une charge matifiante, le distributeur permettant de délivrer au moins ces deux produits de base dans des proportions réglables.
lxxxvi) Système selon lxxxv), le premier produit de base contenant une huile.
lxxxvii) Système selon lxxxv) ou lxxxvi), le premier produit de base contenant un pigment interférentiel.
lxxxviii) Système selon lxxxv) à lxxxvii), le premier produit de base contenant un piment à reflet métallique.
lxxxix) Système selon lxxxv) à lxxxviii), la taille D50 en volume de particule du pigment et de la charge étant comprise entre 100 nm et 1 mm.
xc) Système selon lxxxv) à lxxxix), la charge étant choisie parmi le talc, le mica, la silice, le kaolin, les poudres de poly-β-alanine et de polyéthylène, les poudres de polymères de tétrafluoroéthylène, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges.
xci) Système selon lxxxv) à lxxxix), le premier produit de base et le deuxième produit de base comportant une émulsion inverse.
xcii) Système selon lxxxv) à xci), la teneur massique en charge dans le deuxième produit de base étant supérieure ou égale à 0,5% et de préférence à 1%.
xciii) Système selon lxxxv) à xcii), comportant une troisième cartouche avec un troisième produit de base.
xciv) Système selon lxxxv) à xciii), les cartouches étant reçues de façon amovible dans le distributeur.
xcv) Système selon lxxxv) à xciv), chaque produit quittant la cartouche par un canal de sortie de la cartouche, le canal de sortie étant défini par un embout de distribution de la cartouche entrainé en rotation relative par rapport à un corps de la cartouche par un mécanisme d'entraînement du distributeur pour distribuer le produit de base contenu dans la cartouche.
xcvi) Procédé d'application d'un produit cosmétique, de maquillage et/ou de soin, sur les matières kératiniques humaines, à l'aide d'un système de distribution selon lxxxv) à xcv), comportant le réglage du distributeur en fonction de la zone à traiter, et la distribution du produit par prélèvement dans les cartouches des produits de base dans les proportions correspondant au réglage du distributeur.

## Revendications

1. Système (10) de distribution d'un produit de maquillage de la peau, éventuellement selon l'un quelconque des points précédents, comportant un distributeur recevant au moins deux cartouches (30) ayant chacune un réservoir et contenant respectivement des premier et deuxième produits de base, le premier produit de base comportant l'un au moins d'une huile, d'un pigment interférentiel ou à reflet métallique, le deuxième produit de base comportant une charge matifiante, le distributeur permettant de délivrer au moins ces deux produits de base dans des proportions réglables.

2. Système selon la revendication 1, le premier produit de base contenant une huile.

3. Système selon la revendication 1 ou 2, le premier produit de base contenant un pigment interférentiel.

4. Système selon l'une quelconque des revendications précédentes, le premier produit de base contenant un piment à reflet métallique.

5. Système selon l'une quelconque des revendications précédentes, la taille D50 en volume de particule du pigment et de la charge étant comprise entre 100 nm et 1 mm, mieux entre 200 nm et 100 microns, encore mieux entre 200 nm et 50 microns.

6. Système selon l'une quelconque des revendications précédentes, la charge étant choisie parmi le talc, le mica, la silice, le kaolin, les poudres de poly-β-alanine et de polyéthylène, les poudres de polymères de tétrafluoroéthylène, les poudres de nylon, les poudres de polymethacrylate de methyle, la lauroyl-lysine, l'amidon, le nitrure de bore, les microsphères creuses polymériques telles que celles de chlorure de polyvinylidène/acrylonitrile, de copolymères d'acide acrylique, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique, les particules qui ont la forme de portions de sphères creuses, et leurs mélanges, préférentiellement est choisi parmi le talc, le mica, la silice, les poudres de nylon, les poudres de polymethacrylate de methyle, et leurs mélanges, encore préférentiellement est du talc.

7. Système selon l'une quelconque des revendications précédentes, le premier produit de base et le deuxième produit de base comportant une émulsion inverse.

8. Système selon l'une quelconque des revendications précédentes, la teneur massique en charge dans le deuxième produit de base étant supérieure ou égale à 0,5% de la masse du premier produit de base, de préférence supérieure à 1% de la masse du premier produit de base, encore de préférence comprise entre 1% et 5% de la masse du premier produit de base.

9. Système selon l'une quelconque des revendications précédentes, comportant une troisième cartouche avec un troisième produit de base.

10. Système selon l'une quelconque des revendications précédentes, les cartouches étant reçues de façon amovible dans le distributeur.

11. Système selon l'une quelconque des revendications précédentes, chaque produit quittant la cartouche par un canal de sortie de la cartouche, le canal de sortie étant défini par un embout de distribution (56) de la cartouche entrainée en rotation relative par rapport à un corps de la cartouche par un mécanisme d'entraînement du distributeur pour distribuer le produit de base contenu dans la cartouche.

12. Procédé de maquillage de la peau à l'aide d'un système de distribution selon l'une quelconque des revendications précédentes, comportant le réglage du distributeur en fonction de la zone à traiter, et la distribution du produit par prélèvement dans les cartouches des produits de base dans les proportions correspondant au réglage du distributeur.
